# EUROPEAN PATENT SPECIFICATION

(11) **EP 0 946 591 B1**
(45) Date of publication and mention of the grant of the patent: **04.04.2007**
(21) Application number: 97951013.8
(22) Date of filing: 15.12.1997
(51) Int. Cl.: C07K 14/72

(54) **CLONED GLUCAGON-LIKE PEPTIDE-2 RECEPTORS**
KLONIERTE REZEPTOREN DES GLUKAGON-ÄHNLICHEN PEPTIDS-2
RECEPTEURS CLONES DE PEPTIDES-2 SEMBLABLES AU GLUCAGON

(30) Priority: 13.12.1996 US 767224; 24.01.1997 US 787721; 24.04.1997 US 845546
(43) Date of publication of application: 06.10.1999
(73) Proprietor: Allelix Biopharmaceuticals Inc., Mississauga, Ontario, L4V 1V7 (CA)
(72) Inventor: MUNROE, Donald, G., Waterdown, Ontario L0R 2H3 (CA); GUPTA, Ashwani, K., Mississauga, Ontario L5N 6Y4 (CA); VYAS, Tejal, B., Mississauga, Ontario L5B 3K1 (CA); McCALLUM, Kirk, Mississauga, Ontario L5L 1P8 (CA); FAN, Ermi, Toronto, Ontario M6S 4Y8 (CA)
(74) Representative: Horner, Martin Grenville
(86) International application number: PCT/CA1997/000969
(87) International publication number: WO 1998/025955

(56) References cited:
- WO-A-93/19175
- WO-A-95/04821
- WO-A-96/32414
- DRUCKER D J ET AL: "INDUCTION OF INTESTINAL EPITHELIAL PROLIFERATION BY GLUCAGON-LIKE PEPTIDE 2" PROCEEDINGS OF THE NATIONAL ACADEMY OF SCIENCES OF USA, vol. 93, 1990, pages 7911-7916, XP000650124
- MUNROE D.G. ET AL: 'Prototypic G protein-coupled receptor for the intestinotrophic factor glucagon-like peptide 2.' PROC. NATL. ACAD. SCI. USA vol. 96, February 1999, pages 1569 - 1573
- YUSTA B.: 'Enteroendocrine localization of GLP-2 receptor expression in humans and rodents' GASTROENTEROLOGY vol. 119, 2000, pages 744 - 755
- BJERKNES M.; CHENG H.: 'Modulation of specific intestinal epithelial progenitors by enteric neurons.' PNAS EARLY EDITION 2001, pages 1 - 6

### SEQUENCE LISTING

(1) GENERAL INFORMATION
   (i) APPLICANT: ALLELIX BIOPHARMACEUTICALS, INC.
   (ii) TITLE OF THE INVENTION: CLONED GLUCAGON-LIKE PEPTIDE-2 RECEPTORS
   (iii) NUMBER OF SEQUENCES: 28
   (iv) CORRESPONDENCE ADDRESS:
      (A) ADDRESSEE: Osler, Hoskin & Harcourt
      (B) STREET: 50 O'Connor Street
      (C) CITY: Ottawa
      (D) STATE: Ontario
      (E) COUNTRY: Canada K1P 6L2
   (v) COMPUTER READABLE FORM:
      (A) MEDIUM TYPE: Diskette
      (B) COMPUTER: IBM Compatible
      (C) OPERATING SYSTEM: DOS
      (D) SOFTWARE: FastSEQ Version 2.0
   (vi) CURRENT APPLICATION DATA:
      (A) APPLICATION NUMBER: PCT/CA97/00969
      (B) FILING DATE: 15-DEC-1997
      (C) CLASSIFICATION:
   (viii) ATTORNEY/ACENT INFORMATION:
      (A) NAME: Aitken, David W.
      (B) REGISTRATION NUMBER:
      (C) REFERENCE/DOCKET NUMBER: 12771
         8607-021
   (ix) TELECOMMUNICATION INFORMATION:
      (A) TELEPHONE: 613-235-7234
      (B) TELEFAX: 613-235-2867
(2) INFORMATION FOR SEQ ID NO:1:
   (i) SEQUENCE CHARACTERISTICS:
      (A) LENGTH: 2575 base pairs
      (B) TYPE: nucleic acid
      (C) STRANDEDNESS: single
      (D) TOPOLOGY: linear
   (ii) MOLECULE TYPE: DNA
   (ix) FEATURE:
      (A) NAME/KEY: Coding Sequence
      (B) LOCATION: 137...1786
      (D) OTHER INFORMATION:
   (xi) SEQUENCE DESCRIPTION: SEQ ID NO:1:
   AAGCTTCGCG GCCTCTGCAG AKGACTTGTG CAAACACTTC CTCTCTGGAC AAGGAGGAAT      60
   GCAGGAGGCC ACCGCCTGCA GTACATCTTG GAGTGTTGGA GGGATGTGCC TGCACTTGTG     120
   AACGGGCGCC AGGAGA ATG AGG CCC CAA CCA AGC CCG GCA GTG CCC AGT AGA    172
                     Met Arg Pro Gln Pro Ser Pro Ala Val Pro Ser Arg
                      1               5                  10
   TGC AGA GAG GCA CCC GTG CCC CGA GTG AGG GCA CAG CCA GTG GGC ATC      220
   Cys Arg Glu Ala Pro Val Pro Arg Val Arg Ala Gln Pro Val Gly Ile
           15                  20                  25
   CCT GAG GCC CAG GGG CCC GTT CCT CTC CAC TCC CAA CAG ATG CGT CTG     268
   Pro Glu Ala Gln Gly Pro Val Pro Leu His Ser Gln Gln Met Arg Leu
       30                  35                  40
   CTG TGG GGC CCT GGG AGG CCC TTC CTC GCC CTG CTT CTG CTG GTT TCC     316
   Leu Trp Gly Pro Gly Arg Pro Phe Leu Ala Leu Leu Leu Leu Val Ser
   45                  50                  55                  60
   ATC AAG CAA GTT ACA GGA TCG CTC CTC AAG GAG ACA ACT CAG AAG TGG     364
   Ile Lys Gln Val Thr Gly Ser Leu Leu Lys Glu Thr Thr Gln Lys Trp
                   65                  70                  75
   GCT AAT TAT AAG GAG AAG TGT CTG GAA GAC TTG CAC AAT AGA CTT TCT     412
   Ala Asn Tyr Lys Glu Lys Cys Leu Glu Asp Leu His Asn Arg Leu Ser
               80                  85                  90
   GGC ATA TTT TGT AAT GGG ACA TTT GAT CGG TAT GTG TGC TGG CCT CAT     460
   Gly Ile Phe Cys Asn Gly Thr Phe Asp Arg Tyr Val Cys Trp Pro His
           95                  100                 105
   TCT TAT CCT GGA AAT GTC TCT GTT CCC TGT CCT TCA TAC TTA CCT TGG     508
   Ser Tyr Pro Gly Asn Val Ser Val Pro Cys Pro Ser Tyr Leu Pro Trp
       110                 115                 120
   TGG AAT GCA GAG AGC CCA GGA AGG GCC TAC AGA CAC TGC TTG GCT CAG     556
   Trp Asn Ala Glu Ser Pro Gly Arg Ala Tyr Arg His Cys Leu Ala Gln
   125                 130                 135                 140
   GGG ACT TGG CAG ACG CGA GAG AAC ACC ACA GAT ATT TGG CAG GAT GAA     604
   Gly Thr Trp Gln Thr Arg Glu Asn Thr Thr Asp Ile Trp Gln Asp Glu
                   145                 150                 155
   TCA GAA TGC TCA GAG AAC CAC AGC TTC AGA CAA AAC GTG GAT CAC TAC     652
   Ser Glu Cys Ser Glu Asn His Ser Phe Arg Gln Asn Val Asp His Tyr
               160                 165                 170
   GCC TTG CTA TAC ACC TTG CAG CTG ATG TAC ACT GTG GGC TAC TCC GTG     700
   Ala Leu Leu Tyr Thr Leu Gln Leu Met Tyr Thr Val Gly Tyr Ser Val
           175                 180                 185
   TCT CTC ATC TCC CTC TTC TTG GCT CTT ACA CTC TTC TTG TTC CTT CGA     748
   Ser Leu Ile Ser Leu Phe Leu Ala Leu Thr Leu Phe Leu Phe Leu Arg
       190                 195                 200
   AAA CTG CAT TGC ACA CGC AAT TAC ATC CAC ATG AAC CTG TTC GCT TCG     796
   Lys Leu His Cys Thr Arg Asn Tyr Ile His Met Asn Leu Phe Ala Ser
   205                 210                 215                 220
   TTC ATC CTG AAA GTT CTG GCT GTC CTG GTG AAG GAC ATG GTC TCC CAC     844
   Phe Ile Leu Lys Val Leu Ala Val Leu Val Lys Asp Met Val Ser His
                   225                 230                 235
   AAC TCT TAC TCC AAG AGG CCC GAT GAT GAG AGT GGA TGG ATG TCA TAT     892
   Asn Ser Tyr Ser Lys Arg Pro Asp Asp Glu Ser Gly Trp Met Ser Tyr
               240                 245                 250
   CTG TCA GAG ACA TCC GTC TCC TGT CGC TCC GTC CAG GTC CTC CTG CAC     940
   Leu Ser Glu Thr Ser Val Ser Cys Arg Ser Val Gln Val Leu Leu His
           255                 260                 265
   TAC TTT GTG GGC ACC AAT CAC TTG TGG CTG CTG GTT GAA GGA CTT TAC     988
   Tyr Phe Val Gly Thr Asn His Leu Trp Leu Leu Val Glu Gly Leu Tyr
       270                 275                 280
   CTC CAC ACT CTG CTG GAG CCC ACA GTG TTT CCT GAA AGG CGG CTG TGG    1036
   Leu His Thr Leu Leu Glu Pro Thr Val Phe Pro Glu Arg Arg Leu Trp
   285                 290                 295                 300
   CCC AAG TAC CTG GTG GTG GGT TGG GCC TTC CCC ATG CTG TTT GTT ATT    1084
   Pro Lys Tyr Leu Val Val Gly Trp Ala Phe Pro Met Leu Phe Val Ile
                   305                 310                 315
   CCC TGG GGT TTT GCC CGT GCA CAC CTG GAG AAC ACA CGG TGC TGG GCC    1132
   Pro Trp Gly Phe Ala Arg Ala His Leu Glu Asn Thr Arg Cys Trp Ala
               320                 325                 330
   ACA AAT GGG AAC CTG AAA ATC TGG TGG ATC ATC AGA GGA CCC ATG CTG    1180
   Thr Asn Gly Asn Leu Lys Ile Trp Trp Ile Ile Arg Gly Pro Met Leu
           335                 340                 345
   CTT TGT GTA ACA GTT AAT TTC TTC ATC TTC CTC AAG ATT CTC AAG CTT    1228
   Leu Cys Val Thr Val Asn Phe Phe Ile Phe Leu Lys Ile Leu Lys Leu
       350                 355                 360
   CTC ATT TCT AAG CTC AAA GCT CAT CAG ATG TGC TTC AGA GAC TAC AAA    1276
   Leu Ile Ser Lys Leu Lys Ala His Gln Met Cys Phe Arg Asp Tyr Lys
   365                 370                 375                 380
   TAC AGA TTG GCG AAA TCA ACG TTG CTC CTC ATT CCT TTG TTG GGG GTT    1324
   Tyr Arg Leu Ala Lys Ser Thr Leu Leu Leu Ile Pro Leu Leu Gly Val
                   385                 390                 395
   CAT GAG GTC CTC TTC ACT TTC TTC CCC GAC GAC CAA GTT CAA GGA TTT    1372
   His Glu Val Leu Phe Thr Phe Phe Pro Asp Asp Gln Val Gln Gly Phe
               400                 405                 410
   TCA AAA CGT ATT CGA CTC TTC ATC CAG CTG ACA CTG AGC TCT GTC CAC    1420
   Ser Lys Arg Ile Arg Leu Phe Ile Gln Leu Thr Leu Ser Ser Val His
           415                 420                 425
   GGA TTT CTG GTG GCC TTG CAG TAT GGC TTT GCC AAT GGA GAG GTG AAG    1468
   Gly Phe Leu Val Ala Leu Gln Tyr Gly Phe Ala Asn Gly Glu Val Lys
       430                 435                 440
   GCA GAG CTG CGA AAG TCA TGG GGC CGC TTC TTA TTA GCC CGC CAC TGG    1516
   Ala Glu Leu Arg Lys Ser Trp Gly Arg Phe Leu Leu Ala Arg His Trp
   445                 450                 455                 460
   GGC TGC AGA ACC TGT GTC CTG GGG AAG AAT TTC CGG TTC CTG GGG AAG    1564
   Gly Cys Arg Thr Cys Val Leu Gly Lys Asn Phe Arg Phe Leu Gly Lys
                   465                 470                 475
   TGT TCC AAG AAG CTG TCG GAG GGA GAT GGC TCT GAG ACA CTC CAG AAG    1612
   Cys Ser Lys Lys Leu Ser Glu Gly Asp Gly Ser Glu Thr Leu Gln Lys
               480                 485                 490
   CTG CGG TTC TCC ACA TGC AGC TCA CAC CTG GCC TCT GAG ACC CTG GGA    1660
   Leu Arg Phe Ser Thr Cys Ser Ser His Leu Ala Ser Glu Thr Leu Gly
           495                 500                 505
   GAC GTT GGG GTA CAG CCT CAC AGG GGC CGT GGA GCT TGG CCC CGG GGA    1708
   Asp Val Gly Val Gln Pro His Arg Gly Arg Gly Ala Trp Pro Arg Gly
       510                 515                 520
   AGC AGC CTG TCT GAG AGC AGT GAG GGA GAC TTC ACC CTG GCC AAT ACG    1756
   Ser Ser Leu Ser Glu Ser Ser Glu Gly Asp Phe Thr Leu Ala Asn Thr
   525                 530                 535                 540
   ATG GAG GAG ATT CTG GAA GAG AGT GAG ATC TAAGGCAGGG TCCATCACCG CAG  1809
   Met Glu Glu Ile Leu Glu Glu Ser Glu Ile
                   545                 550
   CTTGGCCACT GARGAMCCAA CCCTARGAAG GATKTTGCCG ARCCCARGGT CCTCCTCTTC   1869
   CTATGTWCTA TMCCCATTTT GATGTGAAGT CTCTCCTGGG TGAMCAASCT CTGTACCAAC   1929
   SARTCTCAGT CCCTCTTGCC CTTGTCACCC TACTACCCCT CCCCCATCAC ACATGTTTTC   1989
   CAGAATKTCC GTTGGTTTGG GGGGGGGGGT CTTGCCCTAA ATTCAAGTSG AGTGGARCCC   2049
   ACCATGAAGA AAARTCATTT ATTAAATAGA RTCCGGTTAG GATCTCCTTC CCGTTCATGG   2109
   TGCATGGCCT CCTTCCAAGG GATGGGAGTC GGSTGCACTG GAACCCCACA GGAAACTTTG   2169
   AAGTATCCAG TTCTAGGGAA TTATAGCCAA TATTCTGAGA GAGCAAGTCT GAGATGAGAK   2229
   CCGAGAATWG CAAGTGTWGG ACAWGCATTC AAGGAAACTC CTCACCTTTG GGCGAAACCT   2289
   ATGGCACCAT CGGCATGGAG CAGCTATTMT GCAAYGGCCG CTCACCTGGG ACATACCACT   2349
   CTCCTTGGGC AGGATGTGAC CCCATGTKGT CCCCCAGACT CCTCTCCTCC TTGCTTSTST   2409
   TCYTTTCCYG TCAAGTCTCA CCTCCCTTTC TACATCTCAG TTCWGTTTGG TGTYGACAGA   2469
   AGYYTGAATG TCACAATACT GCATGTGTTA GTTTCTGTCG TCATTGCTGT GTCCAAATAC   2529
   CTGACCAGGA CCAATTTAAG CGAGGAACTG CTACATGGGC GGCCGC                  2575
(2) INFORMATION FOR SEQ ID NO:2:
   (i) SEQUENCE CHARACTERISTICS:
      (A) LENGTH: 550 amino acids
      (B) TYPE: amino acid
      (C) STRANDEDNESS:
      (D) TOPOLOGY: unknown
   (ii) MOLECULE TYPE: protein
   (xi) SEQUENCE DESCRIPTION: SEQ ID NO:2:
   Met Arg Pro Gln Pro Ser Pro Ala Val Pro Ser Arg Cys Arg Glu Ala
   1               5                   10                  15
   Pro Val Pro Arg Val Arg Ala Gln Pro Val Gly Ile Pro Glu Ala Gln
               20                  25                  30
   Gly Pro Val Pro Leu His Ser Gln Gln Met Arg Leu Leu Trp Gly Pro
           35                  40                  45
   Gly Arg Pro Phe Leu Ala Leu Leu Leu Leu Val Ser Ile Lys Gln Val
       50                  55                  60
   Thr Gly Ser Leu Leu Lys Glu Thr Thr Gln Lys Trp Ala Asn Tyr Lys
   65                  70                  75                  80
   Glu Lys Cys Leu Glu Asp Leu His Asn Arg Leu Ser Gly Ile Phe Cys
                   85                  90                  95
   Asn Gly Thr Phe Asp Arg Tyr Val Cys Trp Pro His Ser Tyr Pro Gly
               100                 105                 110
   Asn Val Ser Val Pro Cys Pro Ser Tyr Leu Pro Trp Trp Asn Ala Glu
           115                 120                 125
   Ser Pro Gly Arg Ala Tyr Arg His Cys Leu Ala Gln Gly Thr Trp Gln
       130                 135                 140
   Thr Arg Glu Asn Thr Thr Asp Ile Trp Gln Asp Glu Ser Glu Cys Ser
   145                 150                 155                 160
   Glu Asn His Ser Phe Arg Gln Asn Val Asp His Tyr Ala Leu Leu Tyr
                   165                 170                 175
   Thr Leu Gln Leu Met Tyr Thr Val Gly Tyr Ser Val Ser Leu Ile Ser
               180                 185                 190
   Leu Phe Leu Ala Leu Thr Leu Phe Leu Phe Leu Arg Lys Leu His Cys
           195                 200                 205
   Thr Arg Asn Tyr Ile His Met Asn Leu Phe Ala Ser Phe Ile Leu Lys
       210                 215                 220
   Val Leu Ala Val Leu Val Lys Asp Met Val Ser His Asn Ser Tyr Ser
   225                 230                 235                 240
   Lys Arg Pro Asp Asp Glu Ser Gly Trp Met Ser Tyr Leu Ser Glu Thr
                   245                 250                 255
   Ser Val Ser Cys Arg Ser Val Gln Val Leu Leu His Tyr Phe Val Gly
               260                 265                 270
   Thr Asn His Leu Trp Leu Leu Val Glu Gly Leu Tyr Leu His Thr Leu
           275                 280                 285
   Leu Glu Pro Thr Val Phe Pro Glu Arg Arg Leu Trp Pro Lys Tyr Leu
       290                 295                 300
   Val Val Gly Trp Ala Phe Pro Met Leu Phe Val Ile Pro Trp Gly Phe
   305                 310                 315                 320
   Ala Arg Ala His Leu Glu Asn Thr Arg Cys Trp Ala Thr Asn Gly Asn
                   325                 330                 335
   Leu Lys Ile Trp Trp Ile Ile Arg Gly Pro Met Leu Leu Cys Val Thr
               340                 345                 350
   Val Asn Phe Phe Ile Phe Leu Lys Ile Leu Lys Leu Leu Ile Ser Lys
           355                 360                 365
   Leu Lys Ala His Gln Met Cys Phe Arg Asp Tyr Lys Tyr Arg Leu Ala
       370                 375                 380
   Lys Ser Thr Leu Leu Leu Ile Pro Leu Leu Gly Val His Glu Val Leu
   385                 390                 395                 400
   Phe Thr Phe Phe Pro Asp Asp Gln Val Gln Gly Phe Ser Lys Arg Ile
                   405                 410                 415
   Arg Leu Phe Ile Gln Leu Thr Leu Ser Ser Val His Gly Phe Leu Val
               420                 425                 430
   Ala Leu Gln Tyr Gly Phe Ala Asn Gly Glu Val Lys Ala Glu Leu Arg
           435                 440                 445
   Lys Ser Trp Gly Arg Phe Leu Leu Ala Arg His Trp Gly Cys Arg Thr
       450                 455                 460
   Cys Val Leu Gly Lys Asn Phe Arg Phe Leu Gly Lys Cys Ser Lys Lys
   465                 470                 475                 480
   Leu Ser Glu Gly Asp Gly Ser Glu Thr Leu Gln Lys Leu Arg Phe Ser
                   485                 490                 495
   Thr Cys Ser Ser His Leu Ala Ser Glu Thr Leu Gly Asp Val Gly Val
               500                 505                 510
   Gln Pro His Arg Gly Arg Gly Ala Trp Pro Arg Gly Ser Ser Leu Ser
           515                 520                 525
   Glu Ser Ser Glu Gly Asp Phe Thr Leu Ala Asn Thr Met Glu Glu Ile
       530                 535                 540
   Leu Glu Glu Ser Glu Ile
   545                 550
(2) INFORMATION FOR SEQ ID NO:3:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:3:
      TTTTTCTAGA ASRTSATSTA CACNGTSGGC TAC 33
(2) INFORMATION FOR SEQ ID NO:4:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:4:
      TTTTCTCGAG CCARCARCCA SSWRTARTTG GC 32
(2) INFORMATION FOR SEQ ID NO:5:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:5:
      AACTACATCC ACMKGMAYCT GTTYVYGTCB TTCATSCT 38
(2) INFORMATION FOR SEQ ID NO:6:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:6:
      TCATCTCCCT CTTCTTGGCT CTTAC 25
(2) INFORMATION FOR SEQ ID NO:7:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:7:
      TCTGACAGAT ATGACATCCA TCCAC 25
(2) INFORMATION FOR SEQ ID NO:8:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:8:
      TCYRNCTGSA CCTCMYYRTT GASRAARCAG TA 32
(2) INFORMATION FOR SEQ ID NO:9:
   (i) SEQUENCE CHARACTERISTICS:
      (A) LENGTH: 667 base pairs
      (B) TYPE: nucleic acid
      (C) STRANDEDNESS: single
      (D) TOPOLOGY: linear
   (ii) MOLECULE TYPE: DNA
   (ix) FEATURE:
      (A) NAME/KEY: Coding Sequence
      (B) LOCATION: 1...664
      (D) OTHER INFORMATION:
   (xi) SEQUENCE DESCRIPTION: SEQ ID NO:9:
   TCC TTC TCT CTT ATC TCC CTC TTC CTG GCT CTC ACC CTC CTC TTG TTT      48
   Ser Phe Ser Leu Ile Ser Leu Phe Leu Ala Leu Thr Leu Leu Leu Phe
    1               5                  10                  15
   CTT CGA AAA CTC CAC TGC ACG CGC AAC TAC ATC CAC ATG AAC TTG TTT      96
   Leu Arg Lys Leu His Cys Thr Arg Asn Tyr Ile His Met Asn Leu Phe
               20                  25                  30
   GCT TCT TTC ATC CTG AGA ACC CTG GCT GTA CTG GTG AAG GAC GTC GTC     144
   Ala Ser Phe Ile Leu Arg Thr Leu Ala Val Leu Val Lys Asp Val Val
           35                  40                  45
   TTC TAC AAC TCT TAC TCC AAG AGG CCT GAC AAT GAG AAT GGG TGG ATG     192
   Phe Tyr Asn Ser Tyr Ser Lys Arg Pro Asp Asn Glu Asn Gly Trp Met
       50                  55                  60
   TCC TAC CTG TCA GAG ATG TCC ACC TCC TGC CGC TCA GTC CAG GTT CTC     240
   Ser Tyr Leu Ser Glu Met Ser Thr Ser Cys Arg Ser Val Gln Val Leu
   65                  70                  75                  80
   TTG CAT TAC TTT GTG GGT GCC AAT TAC TTA TGG CTG CTG GTT GAA GGC     288
   Leu His Tyr Phe Val Gly Ala Asn Tyr Leu Trp Leu Leu Val Glu Gly
                   85                  90                  95
   CTC TAC CTC CAC ACG CTG CTG GAG CCC ACA GTG CTT CCT GAG AGG CGG     336
   Leu Tyr Leu His Thr Leu Leu Glu Pro Thr Val Leu Pro Glu Arg Arg
               100                 105                 110
   CTG TGG CCC AGA TAC CTG CTG TTG GGT TGG GCC TTC CCT GTG CTA TTT     384
   Leu Trp Pro Arg Tyr Leu Leu Leu Gly Trp Ala Phe Pro Val Leu Phe
           115                 120                 125
   GTT GTA CCC TGG GGT TTC GCC CGT GCA CAC CTG GAG AAC ACA GGG TGC     432
   Val Val Pro Trp Gly Phe Ala Arg Ala His Leu Glu Asn Thr Gly Cys
       130                 135                 140
   TGG ACA ACA AAT GGG AAT AAG AAA ATC TGG TGG ATC ATC CGA GGA CCC     480
   Trp Thr Thr Asn Gly Asn Lys Lys Ile Trp Trp Ile Ile Arg Gly Pro
   145                 150                 155                 160
   ATG ATG CTC TGT GTA ACA GTC AAT TTC TTC ATC TTC CTG AAA ATT CTC     528
   Met Met Leu Cys Val Thr Val Asn Phe Phe Ile Phe Leu Lys Ile Leu
                   165                 170                 175
   AAG CTT CTC ATT TCT AAG CTC AAA GCT CAT CAA ATG TGC TTC AGA GAT     576
   Lys Leu Leu Ile Ser Lys Leu Lys Ala His Gln Met Cys Phe Arg Asp
               180                 185                 190
   TAT AAA TAC AGA TTG GCA AAA TCA ACA CTG GTC CTC ATT CCT TTA TTG     624
   Tyr Lys Tyr Arg Leu Ala Lys Ser Thr Leu Val Leu Ile Pro Leu Leu
           195                 200                 205
   GGC GTT CAT GAG ATC CTC TTC TCT TTC ATC ACT GAT GAT C AAG           667
   Gly Val His Glu Ile Leu Phe Ser Phe Ile Thr Asp Asp Gln
       210                 215                 220
(2) INFORMATION FOR SEQ ID NO;10:
   (i) SEQUENCE CHARACTERISTICS;
      (A) LENGTH: 222 amino acids
      (B) TYPE: amino acid
      (C) STRANDEDNESS:
      (D) TOPOLOCYT unknown
   (ii) MOLECULE TYPE; protein
   (xi) SEQUENCE DESCRIPTION: SEQ ID NO:10:
(2) INFORMATION FOR SEQ ID NO:11:
   (i) SEQUENCE CHARACTERISTICS:
      (A) LENGTH: 2112 base pairs
      (B) TYPE: nucleic acid
      (C) STRANDEDNESS: single
      (D) TOPOLOGY: linear
   (ii) MOLECULE TYPE: DNA
   (ix) FEATURE:
      (A) NAME/KEY: Coding sequence
      (B) LOCATION: 122...1780
      (D) OTHER INFORMATION:
   (xi) SEQUENCE DESCRIPTION: SEQ ID NO:11:
   TGGAGAGGAT TTGTGCAAAC ATTTCTTCTG TGGACCAAGA GGAATGCAAG AGGAGGCTGC      60
   CTGCGGTGCA TCTTGGACGG CTAGAGAGAT GTACCCCTAC TTGTGAAGGT GCACGAGGAA     120
   G ATG AAG CTG GGA TCG AGC AGG GCA GGG CCT GGG AGA GGA AGC GCG GGA     169
     Met Lys Leu Gly Ser Ser Arg Ala Gly Pro Gly Arg Gly Ser Ala Gly
      1               5                  10                  15
   CTC CTG CCT GGC GTC CAC GAG CTG CCC ATG GGC ATC CCT GCC CCC TGG      217
   Leu Leu Pro Gly Val His Glu Leu Pro Met Gly Ile Pro Ala Pro Trp
               20                  25                  30
   GGG ACC AGT CCT CTC TCC TTC CAC AGG AAG TGC TCT CTC TGG GCC CCT      265
   Gly Thr Ser Pro Leu Ser Phe His Arg Lys Cys Ser Leu Trp Ala Pro
           35                  40                  45
   GGG AGG CCC TTC CTC ACT CTG GTC CTG CTG GTT TCC ATC AAG CAA GTT      313
   Gly Arg Pro Phe Leu Thr Leu Val Leu Leu Val Ser Ile Lys Gln Val
       50                  55                  60
   ACA GGA TCC CTC CTT GAG GAA ACG ACT CGG AAG TGG GCT CAG TAC AAA      361
   Thr Gly Ser Leu Leu Glu Glu Thr Thr Arg Lys Trp Ala Gln Tyr Lys
   65                  70                  75                  80
   CAG GCA TGT CTG AGA GAC TTA CTC AAG GAA CCT TCT GGC ATA TTT TGT      409
   Gln Ala Cys Leu Arg Asp Leu Leu Lys Glu Pro Ser Gly Ile Phe Cys
                   85                  90                  95
   AAC GGG ACA TTT GAT CAG TAC GTG TGT TGG CCT CAT TCT TCT CCT GGA      457
   Asn Gly Thr Phe Asp Gln Tyr Val Cys Trp Pro His Ser Ser Pro Gly
               100                 105                 110
   AAT GTC TCT GTA CCC TGC CCT TCA TAC TTA CCT TGG TGG AGT GAA GAG      505
   Asn Val Ser Val Pro Cys Pro Ser Tyr Leu Pro Trp Trp Ser Glu Glu
           115                 120                 125
   AGC TCA GGA AGG GCC TAC AGA CAC TGC TTG GCT CAG GGG ACT TGG CAG     553
   Ser Ser Gly Arg Ala Tyr Arg His Cys Leu Ala Gln Gly Thr Trp Gln
       130                 135                 140
   ACG ATA GAG AAC GCC ACG GAT ATT TGG CAG GAT GAC TCC GAA TGC TCC     601
   Thr Ile Glu Asn Ala Thr Asp Ile Trp Gln Asp Asp Ser Glu Cys Ser
   145                 150                 155                 160
   GAG AAC CAC AGC TTC AAG CAA AAC GTG GAC CGT TAT GCC TTG CTG TCA     649
   Glu Asn His Ser Phe Lys Gln Asn Val Asp Arg Tyr Ala Leu Leu Ser
                   165                 170                 175
   ACC TTG CAG CTG ATG TAC ACC GTG GGA TAC TCC TTC TCT CTT ATC TCC     697
   Thr Leu Gln Leu Met Tyr Thr Val Gly Tyr Ser Phe Ser Leu Ile Ser
               180                 185                 190
   CTC TTC CTG GCT CTC ACC CTC CTC TTG TTT CTT CGA AAA CTC CAC TGC     745
   Leu Phe Leu Ala Leu Thr Leu Leu Leu Phe Leu Arg Lys Leu His Cys
           195                 200                 205
   ACG CGC AAC TAC ATC CAC ATG AAC TTG TTT GCT TCT TTC ATC CTG AGA     793
   Thr Arg Asn Tyr Ile His Met Asn Leu Phe Ala Ser Phe Ile Leu Arg
       210                 215                 220
   ACC CTG GCT GTA CTG GTG AAG GAC GTC GTC TTC TAC AAC TCT TAC TCC     841
   Thr Leu Ala Val Leu Val Lys Asp Val Val Phe Tyr Asn Ser Tyr Ser
   225                 230                 235                 240
   AAG AGG CCT GAC AAT GAG AAT GGG TGG ATG TCC TAC CTG TCA GAG ATG     889
   Lys Arg Pro Asp Asn Glu Asn Gly Trp Met Ser Tyr Leu Ser Glu Met
                   245                 250                 255
   TCC ACC TCC TGC CGC TCA GTC CAG GTT CTC TTG CAT TAC TTT GTG GGT     937
   Ser Thr Ser Cys Arg Ser Val Gln Val Leu Leu His Tyr Phe Val Gly
               260                 265                 270
   GCC AAT TAC TTA TGG CTG CTG GTT GAA GGC CTC TAC CTC CAC ACG CTG     985
   Ala Asn Tyr Leu Trp Leu Leu Val Glu Gly Leu Tyr Leu His Thr Leu
           275                 280                 285
   CTG GAG CCC ACA GTG CTT CCT GAG AGG CGG CTG TGG CCC AGA TAC CTG    1033
   Leu Glu Pro Thr Val Leu Pro Glu Arg Arg Leu Trp Pro Arg Tyr Leu
       290                 295                 300
   CTG TTG GGT TGG GCC TTC CCT GTG CTA TTT GTT GTA CCC TGG GGT TTC    1081
   Leu Leu Gly Trp Ala Phe Pro Val Leu Phe Val Val Pro Trp Gly Phe
   305                 310                 315                 320
   GCC CGT GCA CAC CTG GAG AAC ACA GGG TGC TGG ACA ACA AAT GGG AAT    1129
   Ala Arg Ala His Leu Glu Asn Thr Gly Cys Trp Thr Thr Asn Gly Asn
                   325                 330                 335
   AAG AAA ATC TGG TGG ATC ATC CGA GGA CCC ATG ATG CTC TGT GTA ACA    1177
   Lys Lys Ile Trp Trp Ile Ile Arg Gly Pro Met Met Leu Cys Val Thr
               340                 345                 350
   GTC AAT TTC TTC ATC TTC CTG AAA ATT CTC AAG CTT CTC ATT TCT AAG    1225
   Val Asn Phe Phe Ile Phe Leu Lys Ile Leu Lys Leu Leu Ile Ser Lys
           355                 360                 365
   CTC AAA GCT CAT CAA ATG TGC TTC AGA GAT TAT AAA TAC AGA TTG GCA    1273
   Leu Lys Ala His Gln Met Cys Phe Arg Asp Tyr Lys Tyr Arg Leu Ala
       370                 375                 380
   AAA TCA ACA CTG GTC CTC ATT CCT TTA TTG GGC GTT CAT GAG ATC CTC    1321
   Lys Ser Thr Leu Val Leu Ile Pro Leu Leu Gly Val His Glu Ile Leu
   385                 390                 395                 400
   TTC TCT TTC ATC ACT GAT GAT CAA GTT GAA GGA TTT GCA AAA CTT ATA    1369
   Phe Ser Phe Ile Thr Asp Asp Gln Val Glu Gly Phe Ala Lys Leu Ile
                   405                 410                 415
   CGA CTT TTC ATT CAG TTG ACA CTG AGC TCC TTT CAT GGG TTC CTG GTG    1417
   Arg Leu Phe Ile Gln Leu Thr Leu Ser Ser Phe His Gly Phe Leu Val
               420                 425                 430
   GCC TTG CAG TAT GGT TTT GCC AAT GGA GAA GTG AAG GCT GAG CTG CGG    1465
   Ala Leu Gln Tyr Gly Phe Ala Asn Gly Glu Val Lys Ala Glu Leu Arg
           435                 440                 445
   AAA TAC TGG GTC CGC TTC TTG CTA GCC CGC CAC TCA GGC TGC AGA GCC    1513
   Lys Tyr Trp Val Arg Phe Leu Leu Ala Arg His Ser Gly Cys Arg Ala
       450                 455                 460
   TGT GTC CTG GGG AAG GAC TTC CGG TTC CTA GGA AAA TGT CCC AAG AAG    1561
   Cys Val Leu Gly Lys Asp Phe Arg Phe Leu Gly Lys Cys Pro Lys Lys
   465                 470                 475                 480
   CTC TCG GAA GGA GAT GGC GCT GAG AAG CTT CGG AAG CTG CAG CCC TCA    1609
   Leu Ser Glu Gly Asp Gly Ala Glu Lys Leu Arg Lys Leu Gln Pro Ser
                   485                 490                 495
   CTT AAC AGT GGG CGG CTC CTA CAT CTA GCC ATG CGA GGT CTT GGG GAG    1657
   Leu Asn Ser Gly Arg Leu Leu His Leu Ala Met Arg Gly Leu Gly Glu
               500                 505                 510
   CTG GGC GCC CAG CCC CAA CAG GAC CAT GCA CGC TGG CCC CGG GGC AGC    1705
   Leu Gly Ala Gln Pro Gln Gln Asp His Ala Arg Trp Pro Arg Gly Ser
           515                 520                 525
   AGC CTG TCC GAG TGC AGT GAG GGG GAT GTC ACC ATG GCC AAC ACC ATG    1753
   Ser Leu Ser Glu Cys Ser Glu Gly Asp Val Thr Met Ala Asn Thr Met
       530                 535                 540
   GAG GAG ATT CTG GAA GAG AGT GAG ATC TAGGGTGGAG TTCCACCACC CTGGCTC  1807
   Glu Glu Ile Leu Glu Glu Ser Glu Ile
   545                 550
   TGCTCCCAGG GACTCTTGAG GGGGCCCAGG AAGAGGAAGC AAAGCAGGAC ACACGTTGCT  1867
   GGGCACGGAA TCATTCTCGT TCCATTCACC ATGCCACTTT GATATGAAAG CTATCACAAG  1927
   GTTCTTCAAG CTCTGTATGA AAGAGGCTGT GTGTCATGCT CACAGCCTCT GCCTGCTCTT  1987
   CTCATCCTAA TAACCCCCAC CAGTGTGTTT TCCACAATGC CCACCAGACC CTAGGGCCTG  2047
   GCTCTAAATT CAAGCCAATG AAGTCCCACC CGGAATTCTT TTGCTTTTTA CCCCTGGAAG  2107
   AAATA                                                              2112
(2) INFORMATION FOR SEQ ID NO:12:
   (i) SEQUENCE CHARACTERISTICS:
      (A) LENGTH: 553 amino acids
      (B) TYPE: amino acid
      (C) STRANDEDNESS:
      (D) TOPOLOGY: unknown
   (ii) MOLECULE TYPE: protein
   (xi) SEQUENCE DESCRIPTION: SEQ ID NO:12:
   Met Lys Leu Gly Ser Ser Arg Ala Gly Pro Gly Arg Gly Ser Ala Gly
    1               5                  10                  15
   Leu Leu Pro Gly Val His Glu Leu Pro Met Gly Ile Pro Ala Pro Trp
               20                  25                  30
   Gly Thr Ser Pro Leu Ser Phe His Arg Lys Cys Ser Leu Trp Ala Pro
           35                  40                  45
   Gly Arg Pro Phe Leu Thr Leu Val Leu Leu Val Ser Ile Lys Gln Val
       50                  55                  60
   Thr Gly Ser Leu Leu Glu Glu Thr Thr Arg Lys Trp Ala Gln Tyr Lys
   65                  70                  75                  80
   Gln Ala Cys Leu Arg Asp Leu Leu Lys Glu Pro Ser Gly Ile Phe Cys
                   85                  90                  95
   Asn Gly Thr Phe Asp Gln Tyr Val Cys Trp Pro His Ser Ser Pro Gly
               100                 105                 110
   Asn Val Ser Val Pro Cys Pro Ser Tyr Leu Pro Trp Trp Ser Glu Glu
           115                 120                 125
   Ser Ser Gly Arg Ala Tyr Arg His Cys Leu Ala Gln Gly Thr Trp Gln
       130                 135                 140
   Thr Ile Glu Asn Ala Thr Asp Ile Trp Gln Asp Asp Ser Glu Cys Ser
   145                 150                 155                 160
   Glu Asn His Ser Phe Lys Gln Asn Val Asp Arg Tyr Ala Leu Leu Ser
                   165                 170                 175
   Thr Leu Gln Leu Met Tyr Thr Val Gly Tyr Ser Phe Ser Leu Ile Ser
               180                 185                 190
   Leu Phe Leu Ala Leu Thr Leu Leu Leu Phe Leu Arg Lys Leu His Cys
           195                 200                 205
   Thr Arg Asn Tyr Ile His Met Asn Leu Phe Ala Ser Phe Ile Leu Arg
       210                 215                 220
   Thr Leu Ala Val Leu Val Lys Asp Val Val Phe Tyr Asn Ser Tyr Ser
   225                 230                 235                 240
   Lys Arg Pro Asp Asn Glu Asn Gly Trp Met Ser Tyr Leu Ser Glu Met
                   245                 250                 255
   Ser Thr Ser Cys Arg Ser Val Gln Val Leu Leu His Tyr Phe Val Gly
               260                 265                 270
   Ala Asn Tyr Leu Trp Leu Leu Val Glu Gly Leu Tyr Leu His Thr Leu
           275                 280                 285
   Leu Glu Pro Thr Val Leu Pro Glu Arg Arg Leu Trp Pro Arg Tyr Leu
       290                 295                 300
   Leu Leu Gly Trp Ala Phe Pro Val Leu Phe Val Val Pro Trp Gly Phe
   305                 310                 315                 320
   Ala Arg Ala His Leu Glu Asn Thr Gly Cys Trp Thr Thr Asn Gly Asn
                   325                 330                 335
   Lys Lys Ile Trp Trp Ile Ile Arg Gly Pro Met Met Leu Cys Val Thr
               340                 345                 350
   Val Asn Phe Phe Ile Phe Leu Lys Ile Leu Lys Leu Leu Ile Ser Lys
           355                 360                 365
   Leu Lys Ala His Gln Met Cys Phe Arg Asp Tyr Lys Tyr Arg Leu Ala
       370                 375                 380
   Lys Ser Thr Leu Val Leu Ile Pro Leu Leu Gly Val His Glu Ile Leu
   385                 390                 395                 400
   Phe Ser Phe Ile Thr Asp Asp Gln Val Glu Gly Phe Ala Lys Leu Ile
                   405                 410                 415
   Arg Leu Phe Ile Gln Leu Thr Leu Ser Ser Phe His Gly Phe Leu Val
               420                 425                 430
   Ala Leu Gln Tyr Gly Phe Ala Asn Gly Glu Val Lys Ala Glu Leu Arg
           435                 440                 445
   Lys Tyr Trp Val Arg Phe Leu Leu Ala Arg His Ser Gly Cys Arg Ala
       450                 455                 460
   Cys Val Leu Gly Lys Asp Phe Arg Phe Leu Gly Lys Cys Pro Lys Lys
   465                 470                 475                 480
   Leu Ser Glu Gly Asp Gly Ala Glu Lys Leu Arg Lys Leu Gln Pro Ser
                   485                 490                 495
   Leu Asn Ser Gly Arg Leu Leu His Leu Ala Met Arg Gly Leu Gly Glu
               500                 505                 510
   Leu Gly Ala Gln Pro Gln Gln Asp His Ala Arg Trp Pro Arg Gly Ser
           515                 520                 525
   Ser Leu Ser Glu Cys Ser Glu Gly Asp Val Thr Met Ala Asn Thr Met
       530                 535                 540
   Glu Glu Ile Leu Glu Glu Ser Glu Ile
   545                 550
(2) INFORMATION FOR SEQ ID NO:13:
   (i) SEQUENCE CHARACTERISTICS:
      (A) LENGTH: 462 amino acids
      (B) TYPE: amino acid
      (C) STRANDEDNESS:
      (D) TOPOLOGY: unknown
   (ii) MOLECULE TYPE: protein
   (xi) SEQUENCE DESCRIPTION: SEQ ID NO:13;
(2) INFORMATION FOR SEQ ID NO:14:
   (i) SEQUENCE CHARACTERISTICS:
      (A) LENGTH: 14 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:14:
      AACCCACTGC TTAC 14
(2) INFORMATION FOR SEQ ID NO:15:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:15:
      CCCAGAATAG AATGACACC 19
(2) INFORMATION FOR SEQ ID NO:16:
   (i) SEQUENCE CHARACTERISTICS:
      (A) LENGTH: 24 base pairs
      (B) TYPE: nucleic acid
      (C) STRANDEDNESS: single
      (D) TOPOLOGY; linear
   (ii) MOLECULE TYPE: DNA
   (xi) SEQUENCE DESCRIPTION: SEQ ID NO:16:
      CAGGGGCCGG TACCTCTCCA CTCC 24
(2) INFORMATION FOR SEQ ID NO:17:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:17:
      TTGGGTCCTC GAGTGGCCAA GCTGCG 26
(2) INFORMATION FOR SEQ ID NO:18:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:18:
      GGGTAGTCGG TACCTCTAGA GCAAGTTCAC CC 32
(2) INFORMATION FOR SEQ ID NO:19:
   (i) SEQUENCE CHARACTERISTICS;
      (A) LENGTH: 31 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:19:
      ATAACAGAGG ATCCTCGAGT ATTTCTTCCA G 31
(2) INFORMATION FOR SEQ ID NO:20:
   (i) SEQUENCE CHARACTERISTICS:
      (A) LENGTH: 42 base pairs
      (B) TYPE: nucleic acid
      (C) STRANDEDNESS: single
      (D) TOPOLOGY; linear
   (ii) MOLECULE TYPE: DNA
   (xi) SEQUENCE DESCRIPTION: SEQ ID NO:20:
      ACAGGCATGT CTGGAAGACT TACTCAAGGA ACCTTCTGGC AT 42
(2) INFORMATION FOR SEQ ID NO:21:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:21:
      ATGCCAGAAG GTTCCTTGAG TAAGTCTTCC AGACATGCCT GT 42
(2) INFORMATION FOR SEQ ID NO:22:
   (i) SEQUENCE CHARACTERISTICS;
      (A) LENGTH: 26 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:22:
      TTCCTCTGTG GTACCAAGAG GAATGC 26
(2) INFORMATION FOR SEQ ID NO:23:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:23:
      GGTGGACTCG AGGTACCGAT CTCACTCTCT TCCAGAATC 39
(2) INFORMATION FOR SEQ ID NO:24:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:24:
      GTGGAGAGGA TTTGTGCAAA CATTTC 26
(2) INFORMATION FOR SEQ ID NO:25:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:25;
      AGAGACATTT CCAGGAGAAG AATGAG 26
(2) INFORMATION FOR SEQ ID NO:26:
   (i) SEQUENCE CHARACTERISTICS:
      (A) LENGTH: 15 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO:26;
(2) INFORMATION FOR SEQ ID NO:27:
   (i) SEQUENCE CHARACTERISTICS:
      (A) LENGTH: 13 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO:27:
   Ser Glu Gly Asp Gly Ser Glu Thr Leu Gln Lys Leu Arg
    1               5                   10
(2) INFORMATION FOR SEQ ID NO:28:
   (i) SEQUENCE CHARACTERISTICS:
      (A) LENGTH: 13 amino acids
      (B) TYPE: amino acid
      (C) STRANDEDNESS:
      (D) TOPOLOGY: unknown
   (ii) MOLECULE TYPE: peptide
   (xi) SEQUENCE DESCRIPTION: SEQ ID NO:28:
   Ser His Asn Ser Ser Lys Arg Pro Asp Asp Glu Ser Gly
    1               5                   10

## Description

### FIELD OF THE INVENTION

The invention is in the field of molecular biology. It relates, more particularly, to cloned glucagon-like peptide 2 receptors and their use in drug screening and related applications.

### BACKGROUND TO THE INVENTION

Glucagon-like peptide-2 (GLP-2) is a 33 amino acid peptide, which is expressed in a tissue determined fashion from the pleiotrophic glucagon gene and is highly related in terms of amino acid sequence to glucagon and Glucagon-like peptide-1 (GLP-1). Mammalian forms of GLP-2 are highly conserved: for example, the human and degu (a south American rodent) forms differ by one and three amino acids respectively from rat GLP-2. Recently it was demonstrated that GLP-2 is an intestinotrophic peptide hormone; when given exogenously, GLP-2 can produce a marked increase in the proliferation of small intestinal epithelium of the test mice (Drucker et al, (1996) PNAS, 93:7911-7961). More recently, GLP-2 has been shown to increase D-Glucose maximal transport rate across the intestinal basolateral membrane (Cheeseman and Tseng: American Journal of Physiology (1996) 271:G477-G482). WO 93/19175 discloses cloning of rat and human GLP-1 receptor.

To accelerate research into gastrointestinal biology and development of drugs useful in the treatment of various medical conditions including gastrointestinal disorders, it would be useful to provide the receptor through which the effects of GLP-2 are mediated.

### SUMMARY OF THE INVENTION

The GLP-2 receptor has now been cloned and characterized. Accordingly, the present invention provides an isolated polynucleotide encoding a GLP-2 receptor, particularly including mammalian forms and homologs thereof such as, in specific embodiments, the rat and human forms. In aspects of the invention, polynucleotide coding for a GLP-2 receptor is utilized for expression to obtain functional receptor protein and, in optionally labelled form, for further gene cloning to identify structurally related receptor proteins. In related aspects of the invention, anti-sense versions of GLP-2 receptor-encoding polynucleotides and fragments thereof are obtained and utilized to regulate GLP-2 receptor expression.

In another of its aspects, the invention provides GLP-2 receptor as a product of recombinant production in a cellular host. In related aspects, there are provided recombinant host cells that express GLP-2 receptor, as well as receptor-bearing membranes derived from such cells, and expression constructs in which polynucleotide coding for the GLP-2 receptor is linked to expression controls functional in the selected host cell.

In another of its aspects, the GLP-2 receptor is utilized in a chemicals screening program to identify GLP-2 receptor ligands. This method comprises the steps of incubating the candidate ligand with a GLP-2 receptor-producing cell of the present invention, or with a membrane preparation derived therefrom, and then measuring whether, or the extent to which, binding has occurred. Using cells that express a GLP-2 receptor coupled functionally to a second messenger system, such binding can be determined indirectly, to reveal ligand against activity, by detecting an appropriate reporter.

In another of its aspects, the invention provides antibodies directed to the GLP-2 receptor, for use for example in diagnostic procedures.

The invention is further described with reference to the following drawings in which:

### BRIEF REFERENCE TO THE DRAWINGS

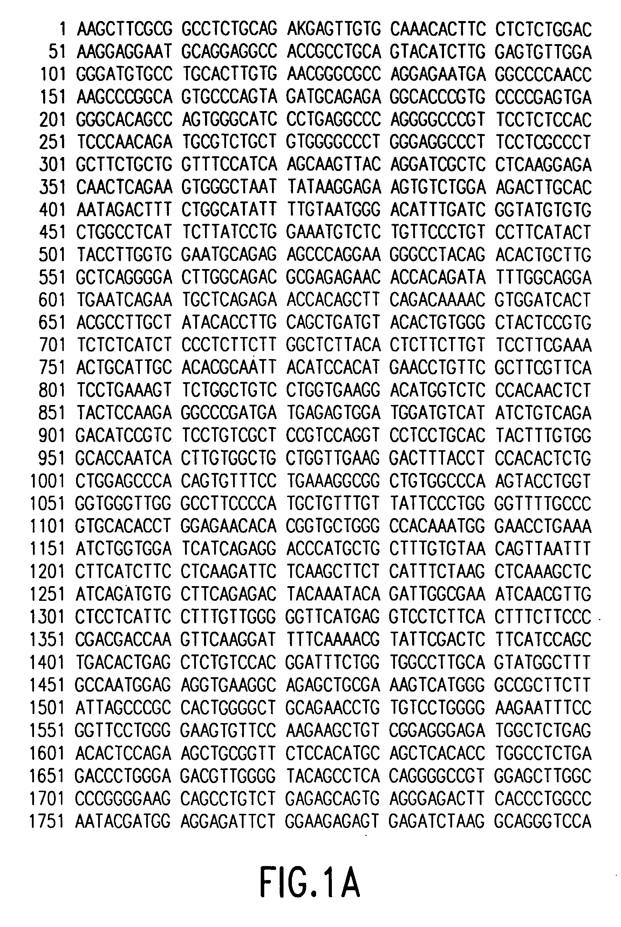

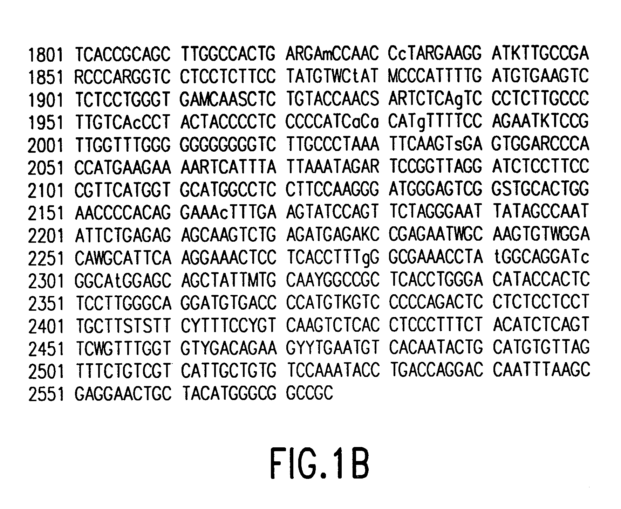

Figure 1 discloses a cDNA sequence (SEQ ID NO: 1), nucleotides 137-1786 of which encode the rat GLP-2 receptor wherein ambiguous base pairs are indicated using the standard IUB nomemenclature (R: A or G, Y: C or T, M: A or C, K: G or T, S: G or C, W: A or T).
Figure 2 discloses the amino acid sequence of the expression product (SEQ ID NO: 2) from the cDNA of Figure 1.
Figure 3 illustrates the relative potencies of GLP-2 peptide and GLP-1 peptides for the receptor encoded by SEQ ID NO: 1.

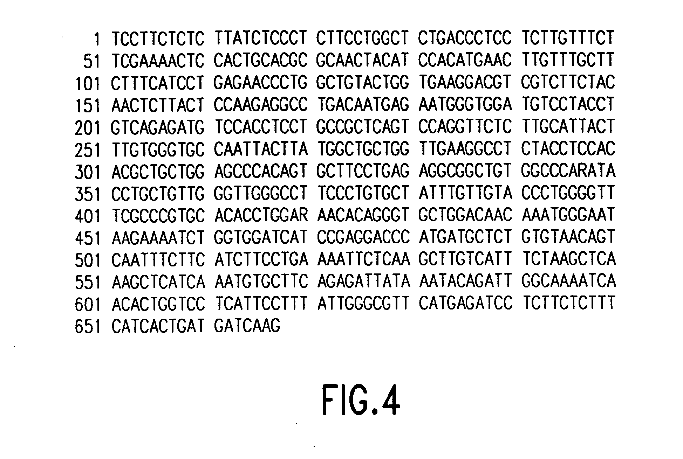

Figure 4 discloses a cDNA sequence of 667 nucleotides (SEQ ID NO: 9) which encodes a 222 amino acid fragment (SEQ ID NO: 10) of a human GLP-2 receptor.

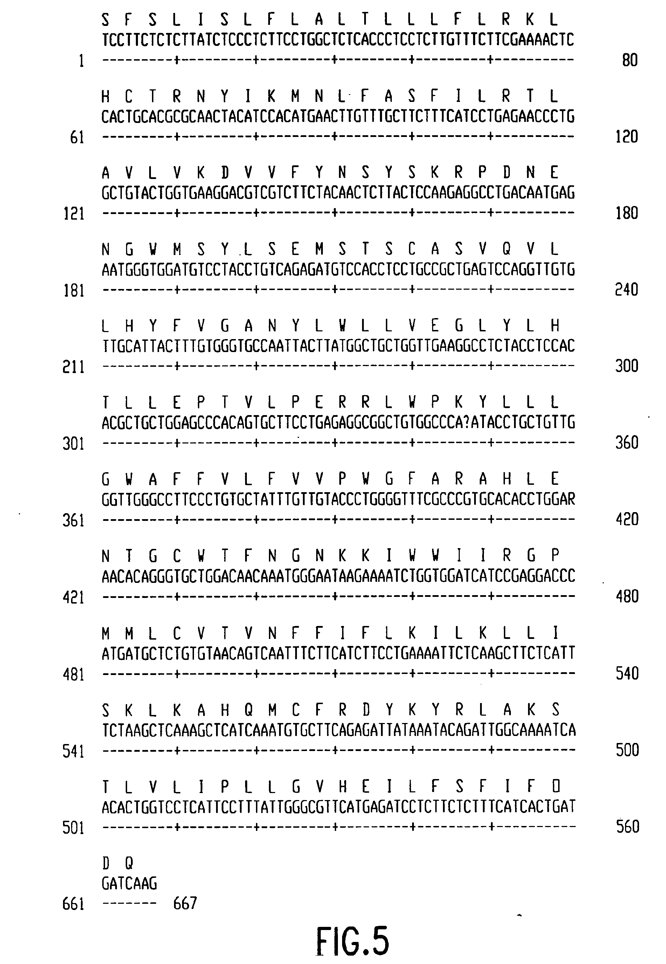

Figure 5 discloses the amino acid sequence (SEQ ID NO: 10) expressed from the cDNA of Figure 4.
Figure 6 discloses a cDNA sequence (SEQ ID NO: 11), nucleotides 122-1780 of which encode a human GLP-2 receptor.
Figure 7 discloses the amino acid sequence of the expression product (SEQ ID NO: 12) from the cDNA of Figure 6.
Figure 8 illustrates the functional activation by GLP-2 peptide of the human receptor encoded by SEQ ID NO: 11 (Figure 8).
Figure 9 compares the amino acid sequences of the rat GLP-2 receptor and the human GLP-2 receptor.
Figure 10 compares the amino acid sequences of the rat GLP-2 receptor and the human GLP-2 receptor against rat GLP-1 receptor.

### DETAILED DESCRIPTION AND PREFERRED EMBODIMENTS

The invention relates in one respect to polynucleotides, in their isolated form, that code for GLP-2 receptors. As used herein "isolated" means separated from polynucleotides that encode other proteins. In the context of polynucleotide libraries, for instance, GLP-2 receptor-encoding polynucleotide is considered "isolated" when it has been selected, and hence removed from association with other polynucleotides within the library. Such polynucleotides may be in the form of RNA, or in the form of DNA including cDNA, genomic DNA and synthetic DNA. The GLP-2 receptors are characterized by structural features common to the G-protein coupled receptor class, including seven transmembrane regions, and by the functional properties of binding GLP-2 peptide selectively, i.e., preferentially, to GLP-1 peptide. Such selective binding is revealed as a statistically significant greater binding affinity of GLP-2 than of GLP-1, in the context of the assay chosen to measure such affinity. When expressed functionally in a host cell, i.e., in operable linkage with a responsive second messenger system the GLP-2 receptors are capable further of responding to GLP-2 binding by signal transduction. In this regard, the activity of a G-protein coupled receptor such as a GLP-2 receptor can be measured using any of a variety of appropriate functional assays in which activation of the receptor results in detectable change in the level of some second messenger system, such as adenylate cyclase, calcium mobilization, inositol phospholipid hydrolysis products or guanylyl cyclase.

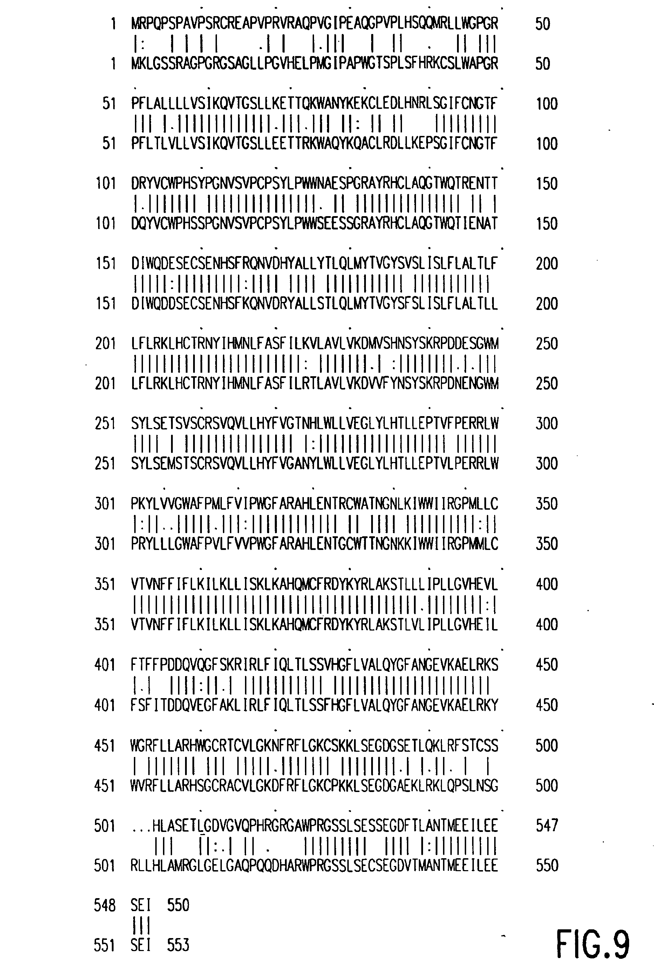

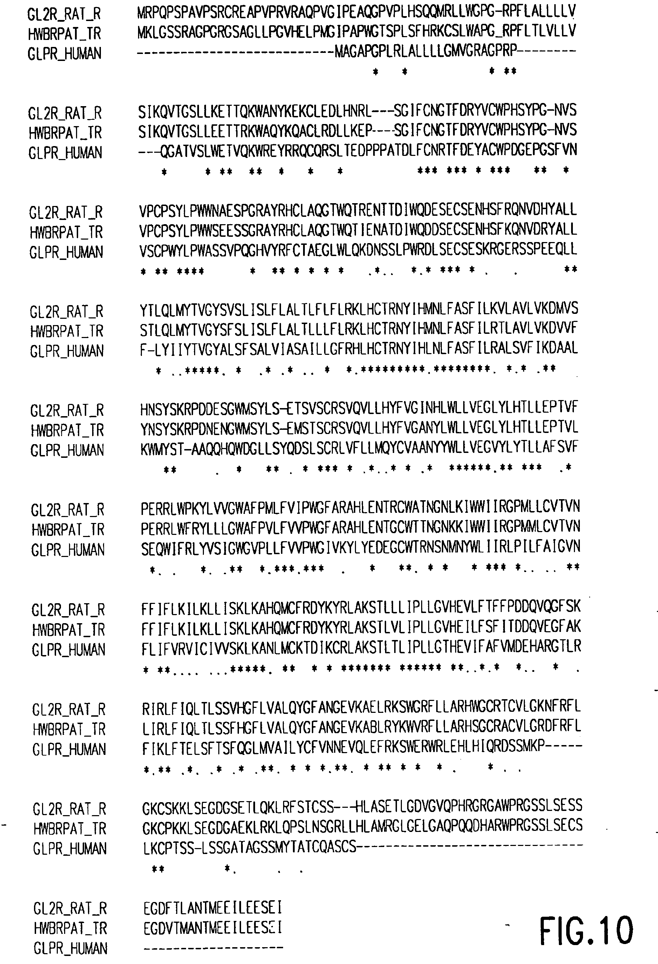

With reference to Figure 9 and Figure 10, which reveals homologies across amino acid sequences representing human and rat GLP-2 receptors, regions of 100% identity are indicated by solid vertical bars. In embodiments of the invention, the GLP-2 receptors are defined structurally as receptors that incorporate these regions of amino acid sequence and that also exhibit the functional characteristic of binding GLP-2 peptide selectively, relative to GLP-1 peptide. In more specific embodiments, the GLP-2 receptor structure further incorporates those amino acids which, across the human and rat receptor species, are highly conserved (indicated by ':'). At these sites, it will be appreciated that the sequence can contain any amino acid within the highly conserved amino acid family to which the identified amino acid belongs. In still more specific embodiments, the GLP-2 receptors have a structure which still further incorporates to the moderatively conserved amino acids (indicated by '.') meaning, at these sites, that the amino acids within the moderately conserved family to which they belong. Beyond these sequences, the GLP-2 receptor structure can vary widely in embodiments of the invention, in allowing for non-conservative amino acid substitutions.

In one embodiment of the invention, the GLP-2 receptor is a rat GLP-2 receptor having the amino acid sequence of SEQ ID NO:2. In a specific embodiment of the invention, this rat GLP-2 receptor is encoded by the polynucleotide sequence of SEQ ID NO: 1. This particular GLP-2 receptor-encoding polynucleotide, also referred to as the WBR gene, is a cDNA of rat origin. The expression product of this polynucleotide incorporates the mature form of the GLP-2 receptor, and additionally incorporates a secretion signal that is removed before membrane integration of the mature GLP-2 receptor product. Such a signal sequence may be naturally present on the polypeptides or replaced with a functionally equivalent secretion signal which is heterologous to the GLP-2 receptor. The replacement secretion signal chosen will depend on the expression system being used and will typically, but not essentially be endogenous to the chosen host and, also typically but not essentially, be homologous to the chosen expression controlling sequences.

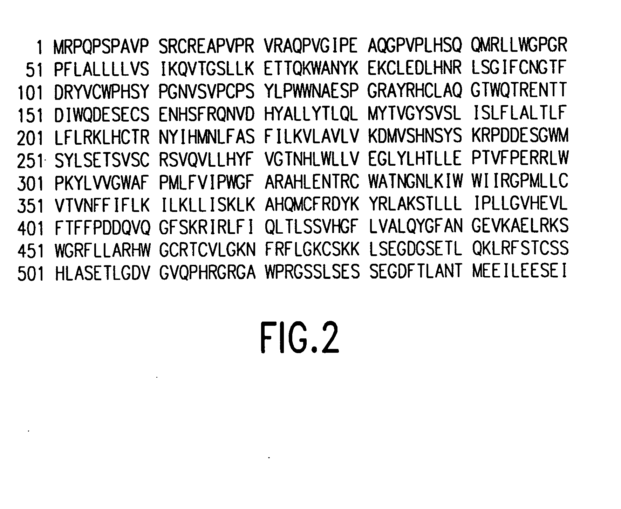

The expressed rat GLP-2 receptor product (Figure 2, SEQ ID NO: 2) is characterized structurally as a single 550 amino acid polypeptide chain having a predicted molecular weight of 72kDa. Two functional translation start sites of the rat GLP-2 receptor have been identified, these are the codons encoding methionine 1 and methionine 42 of SEQ ID NO:2. Without wishing to be limited, by analogy with the GLP-1 receptor, it is believed that residues 1-66 of SEQ ID NO:2 are cleaved to provide a mature protein (i.e., the amino acid sequence of the receptor as it appears in the cell membrane) of 484 amino acids. With respect to structural domains of this GLP-2 receptor, hydropathy analysis and sequence alignment with related members of this sub-family of G protein coupled receptors indicates seven putative transmembrane domains, one spanning residues 181-203 inclusive (TM 1), another spanning residues 211-230 (TM II), a third spanning residues 262-285 (TM III), a fourth spanning residues 300-321 (TM VI), a fifth spanning 339-362 (TM V), a sixth spanning 386-405 (TM VI) and a seventh spanning 422-441 (TM VII). Based on this assignment, it is likely that this GLP-2 receptor, in its natural membrane-bound form, consists of a an N-terminal extracellular domain, followed by a hydrophobic region containing seven transmembrane domains and an intracellular 442-550 amino acid C-terminal domain. The protein exhibits the highest degree of homology to the rat GLP-1 receptor with 49% identity at the amino acid level.

In a related embodiment, the GLP-2 receptor is of human origin (SEQ ID NO: 9) and incorporates the human GLP-2 receptor fragment having the amino acids of SEQ ID NO: 10.

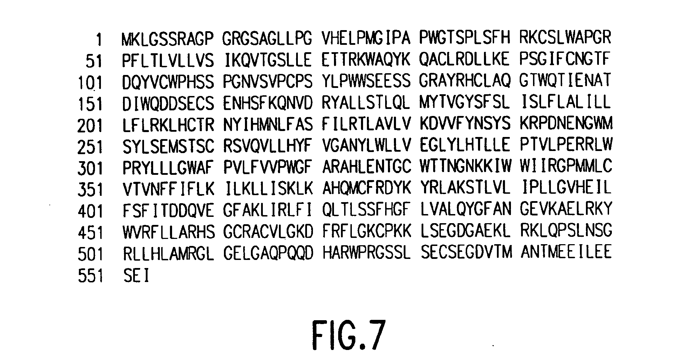

This polynucleotide was isolated using the rat cDNA sequence as generally described below and as detailed in Example 3. In a further related embodiment of the invention, the cDNA is of human origin (SEQ ID NO: 11) and encodes the full length human GLP-2 receptor having residues 67-533 of the amino acid sequence of SEQ ID NO: 12. The human GLP-2 receptor precursor product (Figure 7, SEQ ID NO: 12) is characterized structurally as a single 553 amino acid polypeptide chain having a predicted molecular weight of 72 kDa. It is believed that, as for the rat GLP-2 receptor, this sequence, this precursor form of the human GLP-2 receptor incorporates an N-terminal signal sequence, which can be replaced by a functionally equivalent heterologous signal sequence. Without wishing to be limited it is believed that the mature form of the human GLP-2 receptor results after cleavage of residues 1-66 of SEQ ID NO:12 (Figure 7). With respect to structural domains of this GLP-2 receptor, hydropathy analysis and sequence alignment with related members of this sub-family of G protein coupled receptors indicates seven putative transmembrane domains, one spanning residues 181-203 inclusive (TM I), another spanning residues 211-230 (TM II), a third spanning residues 262-285 (TM III), a fourth spanning residues 300-321 (TM IV), a fifth spanning residues 339-362 (TM V), a sixth spanning residues 386-405 (TM VI), and a seventh spanning residues 422-441 (TM VII). Based on this assignment, it is likely that this GLP-2 receptor, in its natural membrane-bound form, consists of an N-terminal extracellular domain, followed by a hydrophobic region containing seven transmembrane domains interspersed with six short hydrophillic domains, and an intracellular domain, which is predicted to span residues 442-553. A second form of this GLP-2 receptor encompassed by the invention has a translational start site at the methionine codon at position 26 of the amino acid sequence presented in Figure 7 SEQ ID No. 12. The resulting 528 amino acid polypeptide chain also consists of an extracellular domain, seven transmembrane domains, and a C-terminal intracellular domain, and is at least 95% identical in sequence to residues 26-553 of the sequence presented in Figure 7, SEQ ID NO: 12.

In another embodiment, the invention provides GLP-2 receptor polynucleotide sequences and their unique sequence fragments as a tool useful to identify and isolate structurally related polynucleotides. To facilitate isolation of rat GLP-2 receptor gene homologs that are also GLP-2 receptor-encoding, stringency conditions are desirably enhanced to identify homologs having at least 80% (medium stringency) sequence identity homology at the polynucleotide level to receptor gene. More desirably the WBR gene homologs are 90% identical, (high stringency) and most desirably they have at least 95% sequence (high stringency) identity when compared to WBR Preferably, the isolated WBR homologs are characterized in that (1) they can be amplified using the PCR primers of SEQ ID NO: 3 and SEQ ID NO: 4 and (2) they bind to the probe of SEQ ID NO: 5 under high stringency conditions.

Still more preferably, the isolated homologs are those which bind, under conditions of high stringency, with consensus regions of the GLP-2 receptor-encoding polynucleotides, i.e., those regions of the rat and human GLP-2 receptor-encoding polynucleotides which are identical and, relative to GLP-1 receptor-encoding sequences, are also unique to the GLP-2 receptor-encoding polynucleotides. Alignment of this nature reveals a number consensus regions for GLP-2: for example nucleotides spanning from 1460-1786. In one embodiment of the invention, these sequences, and their complements, constitute polynucleotide fragments useful, as just described for the intact gene, to identify polynucleotides structurally related to the human and rat GLP-2 receptor embodiments of the invention.

In a related embodiment, the cDNA sequence or the unique fragments of the GLP-2 receptor, most preferably of the human GLP-2 receptor, can be used in appropriately labelled form, e.g., p³² labelled, for diagnosis of conditions associated with aberrant expression of the GLP-2 receptor. For example, over or under expression or expression in an inappropriate tissue. In one embodiment appropriate PCR primers (e.g., regions unique to the GLP-2 receptor but conserved between species) can be used diagnostically to identify aberrant structure or levels of GLP-2 receptor mRNA, e.g., associated with inherited or acquired disease states.

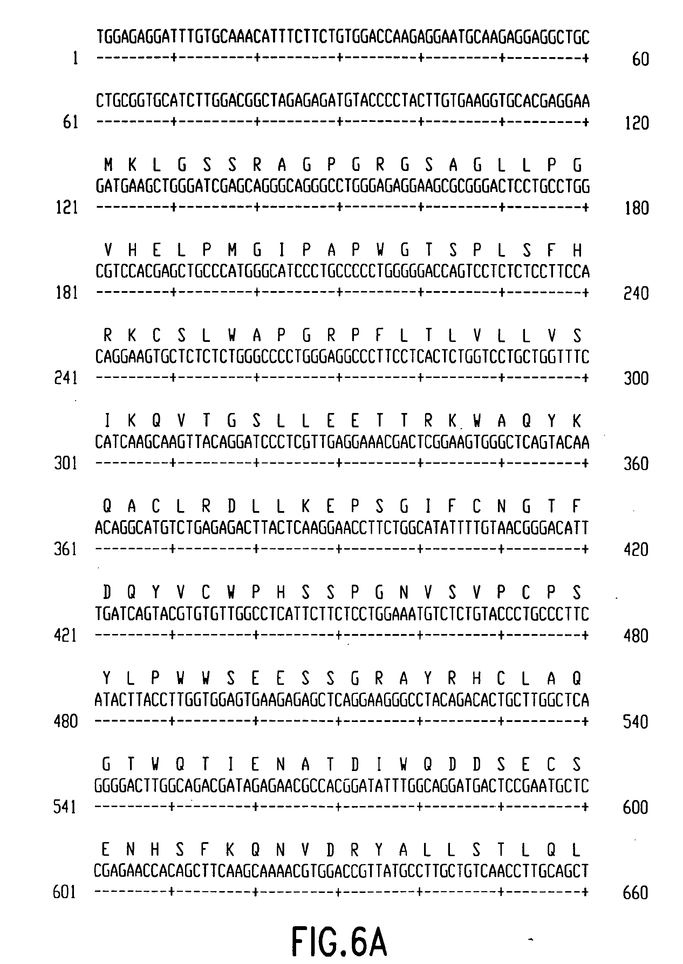

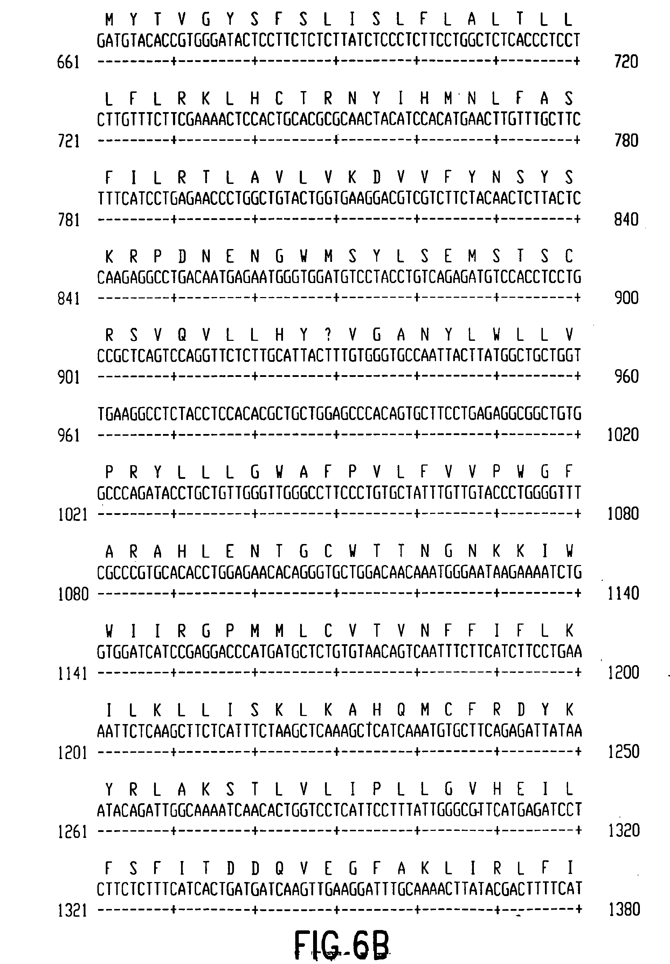

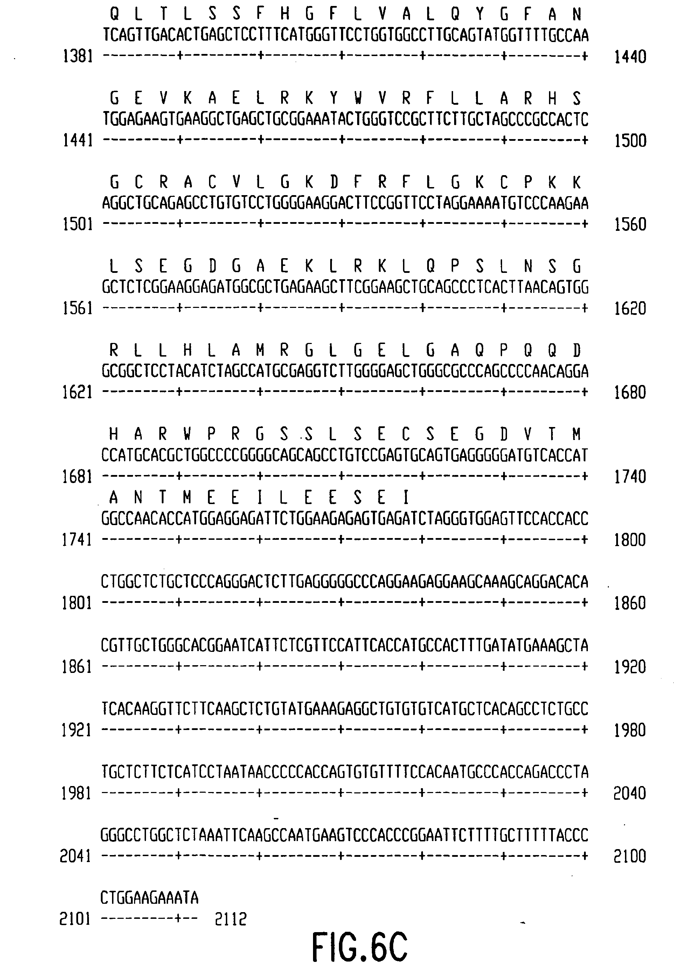

It has been found that the human GLP-2 receptor is located on chromosome 17P13. Thus, in a further embodiment, the invention provides expression products from this locus that hybridize with the human GLP-2 receptor polynucleotide(Figure 6; SEQ ID NO: 11) under stringent conditions.

As source material to isolate GLP-2 receptor-encoding homologs of the rat GLP-2 receptor-encoding polynucleotide gene, it is desirable but not necessary to use libraries of fetal or mature hypothalamal, jejunal, hindbrain or stomach tissue obtained from the vertebrate species targeted for receptor isolation. The invention accordingly includes not only the rat GLP-2 receptor-encoding polynucleotide of SEQ ID NO:1, but structural homologs thereof and particularly those that code for proteins having GLP-2 receptor properties. As exemplified hereinbelow, the WBR gene has been used successfully as a starting material to clone the human homolog of the rat GLP-2 receptor. Thus, the invention provides polynucleotides that encode GLP-2 receptors, including rat GLP-2 receptor and vertebrate homologs, particularly mammalian homologs thereof including human homologs, as well as synthetic variants of these.

It will be appreciated that such homologs can also be identified in libraries by screening, as noted, with fragments of the rat receptor gene or the human homolog, which incorporate at least 15 nucleotides, and preferably at least 25 nucleotides. With reference to SEQ ID NO: 1 and the nucleotide numbering appearing thereon, suitable nucleotide fragments include in addition to the consensus nucleotide fragments noted above, those corresponding in sequence to the extracellular GLP-2 binding domain, and the stipulated transmembrane regions and the C-terminal portion of the receptor.

Technically, the identification of GLP-2 receptor genes can be achieved by applying standard hybridization or amplification techniques to a tissue-derived polynucleotide library. A wide variety of such libraries are commercially available. Where construction of a cDNA library is necessary, established techniques are applied. For example, isolation of such a WBR homolog typically will entail extraction of total messenger RNA from a fresh source of tissue, such as hypothalamal, jejunal, stomach or hindbrain tissue, preferably hypothalamal tissue or cell lines derived from these tissues, followed by conversion of message to cDNA and formation of a library in for example a bacterial plasmid, more typically a bacteriophage.

Such bacteriophage harboring fragments of the DNA are typically grown by plating on a lawn of susceptible E. coli bacteria, such that individual phage plaques or colonies can be isolated. The DNA carried by the phage colony is then typically immobilized on a nitrocellulose or nylon-based hybridization membrane, and then hybridized, under carefully controlled conditions, to a radioactively (or otherwise) labelled probe sequence to identify the particular phage colony carrying the fragment of DNA of particular interest, in this case a rat or human GLP-2 homolog. The phage carrying the particular gene of interest is then purified away from all other phages from the library, in order that the foreign gene may be more easily characterized. Typically, the gene or a portion thereof is then isolated by subcloning into a plasmidic vector for convenience, especially with respect to the full determination of its DNA sequence.

As an alternative to obtaining GLP-2 encoding DNA directly as a DNA insert from an available or a constructed cDNA library, in light of the present disclosure it can be synthesized de novo using established techniques of gene synthesis. Because of the length of the GLP-2 receptor-encoding DNAs of SEQ ID NO: 1, SEQ ID NO: 9 and SEQ ID NO: 11, application of automated synthesis may require staged gene construction, in which regions of the gene up to about 300 nucleotides in length are synthesized individually and then ligated in correct succession for final assembly. Individually synthesized gene regions can be amplified by PCR. The application of automated synthesis may typically be applied by synthesizing specific regions or fragments of the gene and ligating them, usually via designed overlaps, in correct succession to form the final gene sequence. In this case, the longer the oligonucleotide building blocks, the fewer will be the ligations needed, resulting in greater ease of assembly.

The application of automated gene synthesis techniques provides an opportunity for generating sequence variants of the naturally occurring GLP-2 receptor gene. It will be appreciated, for example, that polynucleotides coding for the GLP-2 receptor herein described can be generated by substituting 1 to 20 codons for those represented in the naturally occurring polynucleotide sequences herein provided and such "variants" are within the scope of the present invention. In addition, polynucleotides coding for synthetic variants of the GLP-2 receptor herein provided can be generated which incorporate from 1 to 20, e.g., from 1 to 5, amino acid substitutions, or deletions or additions. Preferred sites for such a modification include areas of non homology between the rat and human sequence, for example amino acid ranges 70-92, 328-350 and 475-504. Since it will be desirable typically to retain the natural ligand binding profile of the receptor for screening purposes, it is desirable to limit amino acid substitutions, for example to the so-called conservative replacements in which amino acids of similar charge are substituted (Fig. 9), and to limit substitutions to those sites less critical for receptor activity. For example, substitution of nucleotides "G" and "A" for nucleotides "A" and "G" respectively at positions 374 and 375 of the human cDNA sequence of SEQ ID NO: 11; resulting in the replacement of the naturally occurring arginine residue at position 85 of SEQ ID NO: 12 with a glutamic acid residue, provides a functional receptor. This functional receptor is referred to herein as the Glu⁸⁵ variant human GLP-2 receptor.

Having obtained GLP-2 receptor encoding polynucleotide, GLP-2 receptor can be produced in a number of ways, including in vitro transcription and via incorporation of the DNA into a suitable expression vector and expression in the appropriate host, for example in a bacterium such as E.coli, in yeast or in insect or in a mammalian cell. A variety of gene expression systems have been adapted for use with these hosts and are now commercially available, and any one of these systems can be selected to drive expression of the GLP-2 receptor-encoding DNA. Expression vectors may be selected to provide transformed cell lines that express the receptor-encoding DNA either transiently or in a stable manner. For transient expression, host cells are typically transformed with an expression vector harboring an origin of replication functional in a mammalian cell. For stable expression, such replication origins are unnecessary, but the vectors will typically harbor a gene coding for a product that confers on the transformants a survival advantage, to enable their selection such as a gene coding for neomycin resistance in which case the transformants are plated in medium supplemented with neomycin.

These expression systems, available typically but not exclusively in the form of plasmidic vectors, incorporate expression cassettes the functional components of which include DNA constituting expression controlling sequences and optionally also signal peptides encoding sequences, which are host-recognized and enable expression of the receptor-encoding DNA when linked 5' thereof. The systems further incorporate DNA sequences which terminate expression when linked 3' of the receptor-encoding region. Thus, for expression in the selected mammalian cell host, there is generated a recombinant DNA expression construct in which the receptor-encoding DNA is linked with expression controlling DNA sequences recognized by the host, and which include a region 5' of the receptor-encoding DNA to drive expression, and a 3' region to terminate expression.

Included among the various recombinant DNA expression systems that can be used to achieve mammalian cell expression of the receptor-encoding DNA are those that exploit promoters of viruses that infect mammalian cells, such as the promoter from the cytomegalovirus (CMV), the Rous sarcoma virus (RSV), simian virus (SV40), murine mammary tumor virus (MMTV) and others. Also useful to drive expression are promoters such as the LTR of retroviruses, insect cell promoters such as those regulated by temperature, and isolated from Drosophila, as well as mammalian gene promoters such as those regulated by heavy metals i.e. the metallothionein gene promoter, and other steroid-inducible promoters.

In another of its aspects, the invention provides cells or membranes derived therefrom which are adapted by genetic alteration for use, for example, in identifying GLP-2 receptor ligands. In preferred embodiments, such cells are adapted genetically by the insertion of polynucleotide coding for a GLP-2 receptor. In particularly preferred embodiments, such cells incorporate a recombinant DNA molecule, e.g. an expression construct/vector, in which DNA coding for the GLP-2 receptor and expression controlling elements functional in the host are linked operably to drive expression of the DNA. For incorporation of receptor into cell plasma membranes, the vector can, if desired, be designed to provide a suitable heterologous signal peptide sequence to substitute for the signal peptide encoded naturally within the receptor DNA.

Suitable GLP-2 producing cells include the Chinese hamster ovary (CHO) cells for example of K1 lineage (ATCC CCL 61) including the Pro5 variant (ATCC CRL 1281); the fibroblast-like cells derived from SV40-transformed African Green monkey kidney of the CV-1 lineage (ATCC CCL 70), of the COS-1 lineage (ATCC CRL 1650) and of the COS-7 lineage (ATCC CRL 1651); murine L-cells, murine 3T3 cells (ATCC CRL 1658), murine C127 cells, human embryonic kidney cells of the 293 lineage (ATCC CRL 1573), human carcinoma cells including those of the HeLa lineage (ATCC CCL 2), and neuroblastoma cells of the lines IMR-32 (ATCC CCL 127), SK-N-MC (ATCC HTB 10) and SK-N-SH (ATCC HTB 11).

For use in ligand screening assays, cell lines expressing the receptor-encoding DNA can be stored frozen for later use. Such assays may be performed either with intact cells, or with membrane preparations derived from such cells. The membrane preparations typically provide a more convenient substrate for the ligand binding experiments, and are therefore preferred as binding substrates. To prepare membrane preparations for screening purpose, i.e., ligand binding experiments, frozen intact cells are homogenized while in cold water suspension and a membrane pellet is collected after centrifugation. The pellet is then washed in cold water, and dialyzed to remove any endogenous GLP-2 receptor ligands that would otherwise compete for binding in the assays. The dialyzed membranes may then be used as such, or after storage in lyophilized form, in the ligand binding assays.

The binding of a candidate ligand to a selected GLP-2 receptor of the invention can be assessed typically using a predetermined amount of cell-derived membrane (measured for example by protein determination), generally from about 25ug to 100ug. Generally, competitive binding assays will be useful to evaluate the affinity of a test compound relative to GLP-2. This competitive binding assay is performed by incubating the membrane preparation with radiolabelled GLP-2 peptide, for example [H³] or a radioiodinated GLP-2 analog, in the presence of unlabelled test compound added at varying concentrations. Following incubation, either displaced or bound radiolabelled GLP-2 can be recovered and measured, to determine the relative binding affinities of the test compound and GLP-2 for the GLP-2 receptor used as substrate. In this way, the affinities of various compounds for the GLP-2 receptor can be measured.

Alternatively, binding of a candidate ligand to a GLP-2 receptor can be assessed using a functional assay. Using this approach, for example, intact cells harvested about two days following transient transfection or after about the same period following plating of stably transfected cells can be used to assess ligand binding. In a preferred embodiment, 293 EBNA cells (Invitrogen Cat. R620-07) are stably transformed with the pREP7 vector (Invitrogen Cat. V007-50) incorporating expressibly therein a GLP-2 receptor. Thereafter, binding of an agonist (or using a competition base format an antagonist) to the receptor can be discerned by measuring the level of intracellular cAMP. Most conveniently, intracellular cAMP is measured indirectly using a reporter system, wherein an easily measurable and preferably easily quantifiable downstream event indicates the level of intracellular cAMP. For example, measuring the level of the expression of a reporter gene construct having polynucleotide sequence under the control of a promoter which is responsive to cAMP. Alternatively, measurement of intracellular calcium, released from intracellular stores in response to an increase in intracellular cAMP, can be used as an indicator of the level of intracellular cAMP, for example, by incorporating into the transformed cell a protein that fluorescences on binding to calcium. In a preferred embodiment, intracellular cAMP levels are measured using the commercially available EIA kit. An additional advantage of the functionally based approach to assessing ligand binding is that the system can be automated allowing high throughput and ultra high through screening of vast chemical libraries.

As an alternative to using cells that express receptor-encoding DNA, ligand characterization may also be performed using cells for example Xenopus oocytes, that yield functional membrane-bound receptor following introduction of messenger RNA coding for the GLP-2 receptor. In this case, the GLP-2 receptor-encoding polynucleotide of the invention is typically subcloned into a plasmidic vector such that the introduced gene may be easily transcribed into RNA via an adjacent RNA transcription promoter supplied by the plasmidic vector, for example the T3 or T7 bacteriophage promoters. RNA is then transcribed from the inserted gene in vitro, and can then be injected into Xenopus oocytes. Each oocyte is a single cell, but is large enough to be penetrated by a fine-tipped microneedle without causing irreparable damage. Following the injection of nL volumes of an RNA solution, the oocytes are left to incubate for up to several days, whereupon the oocytes are tested for the ability to respond to a particular ligand molecule supplied in a bathing solution.

Candidate GLP-2 receptor ligands can vary widely in structure, and most suitably include proteins which are highly related to GLP-2 itself in terms of amino acid sequence. For instance, the peptides disclosed in co-pending United States patent applications WO97/39031 and WO96/32414, may usefully be screened for GLP-2 receptor binding activity.

In addition to naturally occurring GLP-2 receptor sequences functional chimeric, receptors, incorporating portions of the GLP-2 receptor sequence and the polynucleotides encoding them are also embodiments of the invention. Functional chimeric GLP-2 receptors are constructed by combining the extracellular receptive sequences of a GLP-2 receptor with one or more of the transmembrane and intracellular segments of a known seven transmembrane G-protein coupled receptors for test purposes. This concept was demonstrated by Kobilka et al. (1988, Science 240:1310-1316) who created a series of chimeric α2-β2 adrenergic receptors (AR) by inserting progressively greater amounts of α2-AR transmembrane sequence into β2-AR. The binding activity of known agonists changed as the molecule shifted from having more α2 than β2 conformation, and intermediate constructs demonstrated mixed specificity, The specificity for binding antagonists, however, correlated with the source of the transmembrane domain VII. The importance of transmembrane domain VII for ligand recognition was also found in chimeras utilizing two yeast α-factor receptors and is significant because the yeast receptors are classified as miscellaneous receptors. Thus, functional role of specific domains appears to be preserved throughout the seven transmembrane G-protein coupled receptor family regardless of category.

In parallel fashion, internal segments or cytoplasmic domains from a particular GLP-2 receptor are exchanged with the analogs domains of a known seven transmembrane G-protein coupled receptor and used to identify the structural determinants responsible for coupling the receptors to trimeric G-proteins (Dohlman et al. (1991) Annu Rev Biochem 60:653-688). A chimeric receptor in which domains V, VI, and the intracellular connecting loop from β2-AR were substituted into a2-AR was shown to bind ligands with a2-AR specificity, but to stimulate adenylate cyclase in the manner of β2-AR. This demonstrates that for adrenergic-type receptors, G-protein recognition is present in domains V and VI and their connecting loop. The opposite situation was predicted and observed for a chimera in which the V->VI loop from α1-AR replaced the corresponding domain on β2-AR and the resulting receptor bound ligands with β2-AR specificity and activated G-protein-mediated phosphatidylinositol turnover in the α1-AR manner. Finally, chimeras constructed from muscarinic receptors also demonstrated that V->VI loop is the major determinant for specificity of G-protein activity.

Chimeric or modified seven transmembrane G-protein coupled receptors containing substitutions in the extracellular and transmembrane regions have shown that these portions of the receptor determine ligand binding specificity. For example, two Ser residues conserved in domain V of all adrenergic and D catecholamine receptors are necessary for potent agonist activity. These serines are believed to form hydrogen bonds with the catechol moiety of the agonists within the binding site. Similarly, an Asp residue present in domain III of all seven transmembrane G-protein coupled receptors which bind biogenic amines is believed to form an ion pair with the ligand amine group in the binding site.

Functional, cloned seven transmembrane G-protein coupled receptors are expressed in heterologous expression systems and their biological activity assessed (e.g., Marullo et al. (1988) Proc Natl Acad Sci 85:7551-7555; King et al. (1990) Science 250:121-123). One heterologous system introduces genes for a mammalian seven transmembrane G-protein coupled receptors and a mammalian G-protein into yeast cells. The seven transmembrane G-protein coupled receptor is shown to have appropriate ligand specificity and affinity and trigger appropriate biological activation--growth arrest and morphological changes--of the yeast cells.

An alternate procedure for testing chimeric receptors is based on the procedure utilizing the P₂ᵤ purinergic receptor (P₂ᵤ) as published by Erb et al. (1993, Proc Natl Acad Sci 90:104411-104453). Function is easily tested in cultured K562 human leukaemia cells because these cells lack P₂ᵤ receptors. K562 cells are transfected with expression vectors containing either normal or chimeric P₂ᵤ and loaded with fura-a, fluorescent probe for Ca++. Activation of properly assembled and functional P₂ᵤ receptors with extracellular UTP or ATP mobilizes intracellular Ca++ which reacts with fura-a and is measured spectrofluormetrically. As with the seven transmembrane G-protein coupled receptors above, chimeric genes are created by combining sequences for extracellular receptive segments of any newly discovered seven transmembrane G-protein coupled receptors polypeptide with the nucleotides for the transmembrane and intracellular segments of the known P₂ᵤ molecule. Bathing the transfected K562 cells in microwells containing appropriate ligands triggers binding and fluorescent activity defining effectors of the seven transmembrane G-protein coupled receptors molecule. Once ligand and function are established, the P₂ᵤ system is useful for defining antagonists or inhibitors which block binding and prevent such fluorescent reactions.

In addition to using the receptor-encoding DNA to construct cell lines useful for ligand screening, expression of the DNA can according to another aspect of the invention be performed to produce fragments of the receptor in soluble form, for structure investigation, to raise antibodies and for other experimental uses. It is expected that the extracellular portion of the GLP-2 receptor contributes significantly to the binding of ligand molecule. It is therefore desirable in the first instance to facilitate the characterization of the receptor-ligand interaction by providing this extracellular ligand-binding domain in quantity and in isolated form, i.e., free from the remainder of the receptor. Such a construct has been made for the rat GLP-1 receptor, and it was shown to bind GLP-1 (Wilmen et al. (1996) FEBS LETTS, 398:43-47).

To accomplish this, the full-length GLP-2 receptor-encoding DNA may be modified by site-directed mutagenesis, so as to introduce a translational stop codon into the extracellular N-terminal region, immediately before the sequence encoding the first transmembrane domain (TM1), i.e., before residue 181 of SEQ ID NO: 2 and before residue 181 of SEQ ID NO: 12. Since there will no longer be produced any transmembrane domain(s) to "anchor" the receptor into the membrane, expression of the modified gene will result in the secretion, in soluble form, of only the extracellular ligand-binding domain. Standard ligand-binding assays may then be performed to ascertain the degree of binding of a candidate compound to the extracellular domain so produced. It may of course be necessary, using site-directed mutagenesis, to produce several different versions of the extracellular regions, in order to optimize the degree of ligand binding to the isolated domains.

It will be appreciated that the production of such extracellular ligand binding domains may be accomplished in a variety of host cells. Mammalian cells such as CHO cells may be used for this purpose, the expression typically being driven by an expression promoter capable of high-level expression, for example the CMV (cytomegalovirus) promoter. Alternately, non-mammalian cells, such as insect Sf 9 (Spodoptera frugiperda) cells may be used, with the expression typically being driven by expression promoters of the baculovirus, for example the strong, late polyhedron protein promoter. Filamentous fungal expression systems may also be used to secrete large quantities of such extracellular domains of the GLP-2 receptor. Aspergillus nidulans, for example, with the expression being driven by the alcA promoter, would constitute such an acceptable system. In addition to such expression hosts, it will be further appreciated that any prokaryotic or other eukaryotic expression system capable of expressing heterologous genes or gene fragments, whether intracellularly or extracellularly would be similarly acceptable.

The availability of isolated extracellular ligand-binding domains of the receptor protein makes it feasible to determine the 3-dimensional structures of these ligand-binding regions, with or without a candidate ligand complexed thereto, by a combination of X-ray crystallographic and advanced 2D-NMR techniques. In this way, additional new candidate compounds, predicted to have the required interactions with the 3-dimensional receptor structure, can be specifically designed and tested.

With large domains, crystallography is the method of choice for structure determination of both the domain in isolation, and of the co-complex with the natural ligand (or an appropriate antagonist or agonist molecule). If a particular domain can be made small enough, for example approximately 100-130 amino acids in length, then the powerful technique of 2-D NMR can also be applied to structure determination. This enables not only the determination of the domain structure, but also provides dynamic information about the drug-receptor interaction.

For use particularly in detecting the presence and/ or location, for example in intestinal tissue, the present invention also provides, in another of its aspects, labelled antibody to a GLP-2 receptor. To raise such antibodies, there may be used as immunogen either the intact, soluble receptor or an immunogenic fragment thereof, produced in a microbial or mammalian cell host as described above or by standard peptide synthesis techniques. Regions of the GLP-2 receptor particularly suitable for use as immunogenic fragments include those corresponding in sequence to an extracellular region of the receptor, or a portion of the extracellular region, such as peptides consisting of 10 or more amino acids of the 401-509 region of SEQ ID NO: 2. With regard to the human GLP-2 receptor (SEQ ID NO: 12), peptides comprising the mature extracellular domain (residues 65-180); intracellular loop 3 (resides 363-385) and the intracellular C-terminal domain (residues 442-533) may be usefully employed as immunogens for the production of antibodies to the human GLP-2 receptor.

Antibodies to the desired GLP-2 receptor or fragment immunogen are available, for polyclonal antibody production, from the blood of an animal that has been immunized with the immunogen. Alternatively, for monoclonal antibody production, immunocytes-such as splenocytes can be recovered from the immunized animal and fused, using hybridoma technology, to a myeloma cells. The fusion products are then screened by culturing in a selection medium, and cells producing antibody are recovered for continuous growth, and antibody recovery. Recovered antibody can then be coupled covalently to a detectable label, such as a radiolabel, enzyme label, luminescent label or the like, using linker technology established for this purpose.

Animal model systems which elucidate the physiological and behavioral roles of the GLP-2 receptor are produced by creating transgenic animals in which the activity of the GLP-2 receptor is either increased or decreased, or the amino acid sequence of the expressed GLP-2 receptor is altered, by a variety of techniques. Examples of these techniques include, but are not limited to: 1) Insertion of normal or mutant versions of DNA encoding a GLP-2 receptor, by microinjection, electroporation, retroviral transfection or other means well known to those skilled in the art, into appropriate fertilized embryos in order to produce a transgenic animal or 2) Homologous recombination of mutant or normal, human or animal versions of these genes with the native gene locus in transgenic animals to alter the regulation of expression or the structure of these GLP-2 receptor sequences. The technique of homologous recombination is well known in the art. It replaces the native gene with the inserted gene and so is useful for producing an animal that cannot express native GLP-2 receptors but does express, for example, an inserted mutant GLP-2 receptor, which has replaced the native GLP-2 receptor in the animal's genome by recombination, resulting in under expression of the transporter. Microinjection adds genes to the genome, but does not remove them, and so is useful for producing an animal which expresses its own and added GLP-2 receptors, resulting in over expression of the GLP-2 receptors.

One means available for producing a transgenic animal, with a mouse as an example, is as follows: Female mice are mated, and the resulting fertilized eggs are dissected out of their oviducts. The eggs are stored in an appropriate medium such as M2 medium. DNA or cDNA encoding a GLP-2 receptor is cesium chloride purified from a vector by methods well known in the art. Inducible promoters may be fused with the coding region of the DNA to provide an experimental means to regulate expression of the transgene. Alternatively or in addition, tissue specific regulatory elements may be fused with the coding region to permit tissue-specific expression of the trans-gene. The DNA, in an appropriately buffered solution, is put into a microinjection needle (which may be made from capillary tubing using a piper puller) and the egg to be injected is put in a depression slide. The needle is inserted into the pronucleus of the egg, and the DNA solution is injected. The injected egg is then transferred into the oviduct of a pseudopregnant mouse ( a mouse stimulated by the appropriate hormones to maintain pregnancy but which is not actually pregnant), where it proceeds to the uterus, implants, and develops to term. As noted above, microinjection is not the only methods for inserting DNA into the egg cell, and is used here only for exemplary purposes.

The invention having been described above, may be better understood by referring to the following examples. The following examples are offered for the purpose of illustrating the invention and should not be interpreted as a limitation of the invention.

### EXAMPLE 1

### Isolation of the GLP-2 receptor

### PCR-assisted cloning of partial rat and mouse GLP-2 receptor cDNAs

Rat Neonate Intestine cDNA library (Stratagene, La Jolla, CA; Cat. 936508) and Mouse Jejunum first strand cDNA was prepared. Degenerate primers M-2F/S (SEQ ID NO: 3) and M-7R/S (SEQ ID NO: 4) were used to amplify a partial fragment of the rat GLP-2 receptor from the Rat Neonate Intestine cDNA library and of the mouse GLP-2 receptor from Mouse Jejunum template. The protocol is described below:
**Degenerate PCR :**
   6 µl 10x VENT buffer from New England Biolabs
   6 µl 2.5µM each stock dATP, dCTP, dGTP and dTTP
   4 µl rat neonate intestine cDNA (1:10 dilution)
   3 µl 25µM M2F/S primer [5'-TTTTTCTAGAASRTSATSTACACNGTSGGCTAC-3'] (SEQ ID NO: 3)
   3 µl 25µM M7R/S primer [5'-TTTTCTCGAGCCARCARCCASSWRTARTTGGC-3'] (SEQ ID NO: 4)
   2 µl (10 units) Amplitaq DNA polymerase (Perkin Elmer)
   36 µl ddH₂O.
Reaction conditions: 35 cycles at 94°C, 2°min.; 94°C, 1 min.; 53°C, 30 sec.; 72°C, 1 min.
The predominant PCR product was a 303 base pair (bp) DNA fragment. 30 µl samples of the above PCR were purified using the QIAGEN PCR purification kit and eluted in 30 µl ddH₂O. The resulting product was then re-amplified using the same degenerate PCR conditions, with the exception only of 31 cycles at 94°C.

The predominant product at 303 base pair (bp) was cut out and purified using QIAGEN QIAquick gel purification protocol into 30 µl ddH₂O. The resulting product was then re-amplified using the same degenerate PCR conditions, with the exception only of 31 cycles at 94°C.

Next, double digest (Xba I and Xho I) was done on the entire reamplified PCR reaction as follows: 28 µl DNA; 16 µl 10X One-Phor-All buffer (Pharmacia); 2 µl (40 units) Xba I enzyme (Pharmacia); 2 µl (40 units) Xho I enzyme (Pharmacia); and 30 µl ddH₂O.

The samples were digested 4 hours in 37°C water block heater, brought up to 100 µl volume with ddH₂O (sterile) and purified by (1) equal amount (100ul) chloroform extraction; (2) weekend precipitation with 2 volumes ethanol/10 volumes 3M sodium acetate; (3) 1x wash with 70% EtOH; and (4) resuspension in 10 µl 1x TE (pH 8.0).

pBluescript clone 5HT1F#9 was next digested with Xba I and Xho I as follows:
10 µl DNA (pBluescript clone 5HT1F#9)
5 µl 10X NEBuffer 2 (New England Biolabs)
3 µl (1:20 dilution=3 units) Xba I (New England Biolabs)
3 µl (1:20 dilution=3 units) Xho I (New England Biolabs)
5 µl (10x) BSA (New England Biolabs)
24 µl ddH₂O.

The sample was digested for 3 hours in 37 C water block heater, heat-inactivated at 65°C for 20 min and purified using GeneCleanII kit from BIO 101. Aliquots of the PCR reactions were cloned into the above pBluescript plasmid vector using T4 DNA ligase kit (New England Biolabs) and transformed into Epicurean Coli XL-2 Blue MRF' Ultracompetent cells (Stratagene). The transformation was plated onto 2xYT + AMP plates and single colonies were picked. DNA minipreps were made using QIAGEN QIA-prep 8 miniprep kit and the sequences of the fragments were determined using ABI system. Novel sequences were identified containing a partial fragment of the rat and mouse GLP-2 receptor sequence.

Cloning of cDNA with complete GLP-2 receptor coding region was achieved as follows: First, cDNA libraries from the following three tissues were used for screening,
1. Rat Hypothalamus (RHT)
2. Rat Hind Brain (RHB)
3. Rat Duodenum and Jejunum (RDJ)
The three cDNA libraries were prepared by priming with random primer and subcloning unidirectionally into Hind III and Not I sites of pcDNA3.

Next, the three cDNA libraries were homology screened by a degenerate oligo C4-4
[5'-AACTACATCCACMKGMAYCTGTTYVYGTCBTTCATSCT-3'] (SEQ ID NO: 5)
by colony lifts and filter hybridization. The following hybridization conditions were employed: 5X SSPE (1X SSPE is 0.18M NaCl, 10mM NaH₂PO₄ (pH 7.4), 10mM EDTA (pH 7.4)) and 5X Denharts solution (1% Ficoll, 1% Polyvinylpyrrolidone, 1% BSA); 25 mg/ml salmon sperm DNA.

Filters were hybridized at 50°C overnight. Then the filters were washed 2 times in 2X SSPE and 1% SDS at room temperature for 30 min, 2 times in 2X SSPE and 1% SDS at 50°C for 20 min per wash, and finally two times in 1X SSPE and 0.5% SDS. Positive clones were identified by autoradiography. A plug of 1cm² surrounding the positive clone was removed from the plate and placed in 1 ml of 2x YT +20% Glycerol, vortexed and was frozen at -80°C.

Plasmid DNA from positive plugs was prepared as follows: 100 ml of bacterial culture of each positive plug was grown on an agar plate. The bacterial cells were scraped and resuspended in 1 ml of 2xYT medium+20% Glycerol. Bacterial pellet from the 250 ml of bacterial resuspension was resuspended in 150 ml of Solution I (50mM Glucose, 10mM Tris-HCl, 1mM EDTA), lyse in Solution II (0.2M NAOH, 1% SDS), neutralized with ice cold Solution III (Potassium acetate; 4 vol. of 5M potassium acetate + 1 vol. of 10M acetic acid). After pelleting bacterial DNA, 340 ml isopropanol was added to the supernatant. This was centrifuged at max for 15 min. The pellet was resuspended in TE + 20 mg/ml RNase, incubated at 37 C for 30 minutes and precipitated with isopropanol + 0.2M potassium acetate. After centrifugation, the pellet was washed with 70% alcohol, allowed to air dry and resuspended in TE.

Plasmid DNA from 2777-clone pools of rat hypothalamus cDNA library RHT cDNA library was next exploited as follows: Two primers were designed from an area of the PCR-cloned GLP-2 receptor cDNA sequence that did not have identity to known receptors of the gene family. The two primers P23-R1 and P23-F1 amplified a 196 bp fragment only from novel clone DNA but not with GLP-1 receptor cDNA or PACAP receptor cDNA. The Expand^{™} PCR system from Boehringer Mannheim (Cat. 1681-842) was used under the following conditions:
2 µl of 10x Expand^{™} Buffer 1
2.8 µl of 2.5mM dNTP mix
0.6 µl of primer P23-R1 [5'-TCATCTCCCTCTTCTTGGCTCTTAC-3'] (SEQ ID NO: 6)
0.6 µl of primer P23-F1 [5'-TCTGACAGATATGACATCCATCCAC-3'] (SEQ ID NO: 7)
0.3 µl of Expand PCR enzyme (1 unit)
12.7 µl water
1 µl DNA
Reaction conditions: 32 cycles at 93°C, 40 sec; (cycles) 58°C, 40 sec; (cycles) 68°C, 40 sec

DNAs from each positive plug or pool of 2777-clone pools were amplified with specific primers P23-F1 and P23-R1 under the conditions specified above. Out of 1057 C4-4 hybridization-positive plugs and 884 2777-clone pools only five template sources amplified a 196 bp PCR product. These were: (1) Plug 334, (2) Plug 780, (3) RHT pool 233, (4) RHT pool 440, and (5) RHT pool 587.

Amplification of GLP-2R cDNA from the five positive templates was then performed. By using one specific primer (P23-R1 or P23-F1) and one primer based on pcDNA3 vector (Invitrogen) sequence (830F or 1186R), the GLP-2R cDNA insert was directly amplified from clonally impure plugs or 2777-clone pools. The sequences of the vector primers were as follows:
830F: [5'-AACCCACTGCTTAC-3'] (SEQ ID NO:14)
1186R: [5'-CCCAGAATAGAATGACACC-3'] (SEQ ID NO:15)
The PCR was done, under the following conditions just noted, using Expand^{™} PCR system.

The most prominent band was reamplified, purified and sequenced. Based on the amplified sequence obtained, additional primers were designed and new sequencing carried out. In this manner the complete sequences of the GLP-2R cDNA inserts in all five sources of clones were determined. Sequence analysis showed that only pool RHT 440 and pool RHT 587 contain clones with complete coding sequence of GLP-2R and that the two clones were identical (derived from the same cDNA clone).

Because of difficulty in clonally purifying the GLP-2 receptor cDNA clone from the RHT 440 or RHT 587 cDNA library pools, the cDNA was amplified and recloned into pcDNA3. Based on the sequence obtained from RUT 440 and RHT 587, two primers were designed one which primed starting 4bp upstream of the initiation codon and another which primed starting 8bp downstream of the stop codon.
WBR-C5: [5'-CAGGGGCCGGTACCTCTCCACTCC-3'] (SEQ ID NO:16)
WBR-C3: [5'-TTGGGTCCTCGAGTGGCCAAGCTGCG-3'] (SEQ ID NO:17)

The two primers were used to amplify a DNA fragment of approximately 1525 bp fragment under the following PCR conditions using Expand^{™} PCR system from Boehringer Mannheim (Catalogue no. 1681-842).
10 µl of 1Ox Expand^{™} PCR Buffer 1
14 µl of 2.5mM dNTP mix
3.0 µl of Primer 1 (10 µM) (WBR-C5)
3.0 µl of Primer 2 (10 µM) (WBR-C3)
1.5 µl of Enzyme (5 units)
63.5 µl water
5 µl DNA
Reaction conditions: 5 cycles ( 93°C,1 Min; 72°C, 40s; 60°C, 45 sec; 68°C, 2 min) and 25 cycles ( 93°C,1 min; 72°C, 1 min; 68°C, 2 min).

The amplified product was subcloned into Kpn I and Xho I sites of pcDNA3 vector (Invitrogen). Plasmid DNA was prepared using the method described above.

### EXAMPLE 2

### Functional Assay

Cos-1 cells were transfected as described in Analytical Biochemistry, 218:460-463(1994) with Rat clone 587 GLP-2 receptor, cloned human GLP-2 receptor (pC3/HuGL2R-2), or cloned residue 85 variant human GLP-2 receptor (pC3.1/HuGL2R-MH4), pcDNA3. Rat GLP-1 (7-36) amide was used as a control peptide. Solutions used were as follows:
RSC in RPMI 1640 (49ml RPMI + 1ml FCS + 50ul chloroquine, 100mM);
DEAE/RSC Solution: 18.4 ml RSC +1.6 ml DEAE/Dextran (10 mg/ml).

The assay procedure entailed the following:
a) 50 mg of either rat clone 587 GLP-2 receptor, or cloned human GLP-2 receptor, or cloned residue 85 variant human GLP-2 receptor was added (as plasmid pcDNA3) to a 50 ml tube containing six mls. of RSC and incubated at 37°C.
b) Six ml of DEAE/RSC solution was added to each tube and incubated at 37°C for 2 min.
c) 1.5 ml of COS-1 cell suspension (5.5 millions cells) was added to each tube and incubated for 1 hr 45 min at 37°C.
d) Following incubation, the sample was spun for 5 minutes at low speed, washed with DMEM/F12 + 10% FBS twice, and the pellet resuspended in 12.5 ml DMEM/F12 + 10% FBS media.
e) One ml of cell suspension (step d) was added to each well of 6 well plates coated with poly- D-lysine (from Collaborative Biomedical), containing 3 ml of media (0.45 million cells/well).
f) Plates were incubated at 37°C for 3 days.

Treatment of Transfected Cos-1 cells with GLP-1/GLP-2 analog was done as follows:
Solutions : DMEM/F12 (SFM)+ IBMX (3-isobutyl-1-methylxanthin) 0.85mM +0.1% ascorbic acid and 10 um pargyline (all solutions purchased from Sigma). Media was prepared fresh on day of use.
Assay Procedure: The culture media of each well (transfected 6 well plates, cells) was removed, and the wells were washed once with SFM media. Then 2 ml of SFM + IBMX media was added to each well and plates were incubated at 37°C for 10 min. Following incubation, the SFM + IBMX was removed from each well and fresh SFM + IBMX media containing GLP-1/GLP-2 (GLP-1,7-36,amide from Sigma, [Gly2]hGLP-2 from Allelix) concentration 1, 3, 10 and 30 nM were added to the appropriate wells. Plates incubated at 37°C incubator for 30 min. Following incubation, the media were removed from each well. The wells were washed once with 1ml PBS (Phosphate Buffered Saline). Each well was then treated with 1ml cold 95% ethanol: 5mM EDTA (2:1) at 4°C for 1 hr. Cells from each well then were scraped and transferred into individual eppendorf tubes. Tubes were centrifuged for 5 min at 4°C, and the supernatants were transferred to new eppendorf tubes and dried in speed vacuum. Following drying, tubes were reconstituted in 100ul of Na- Acetate and kept at 4°C, 25 µl of this solution used for cAMP Assay.

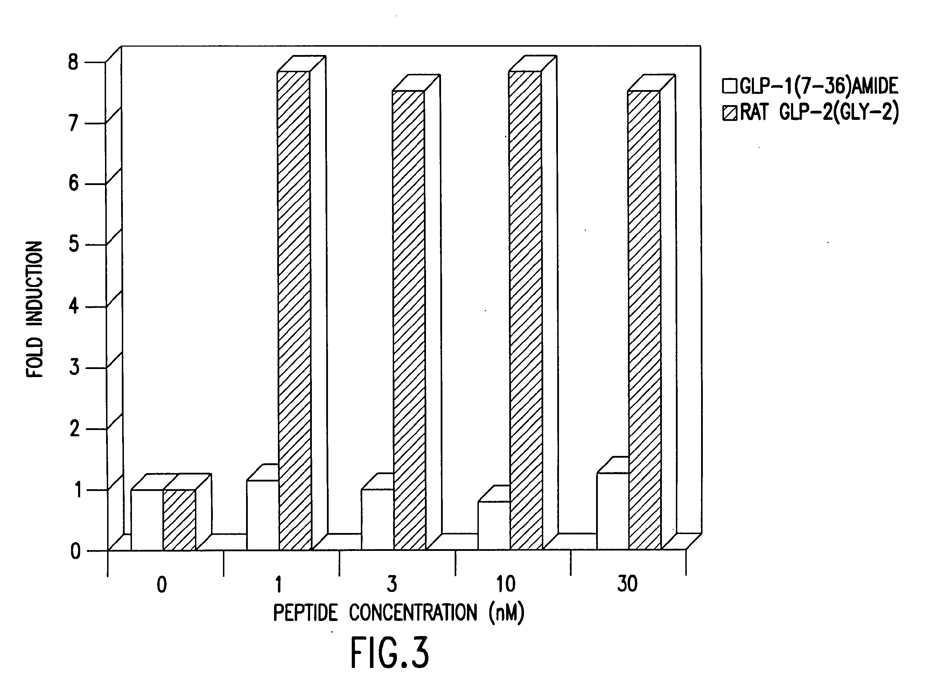

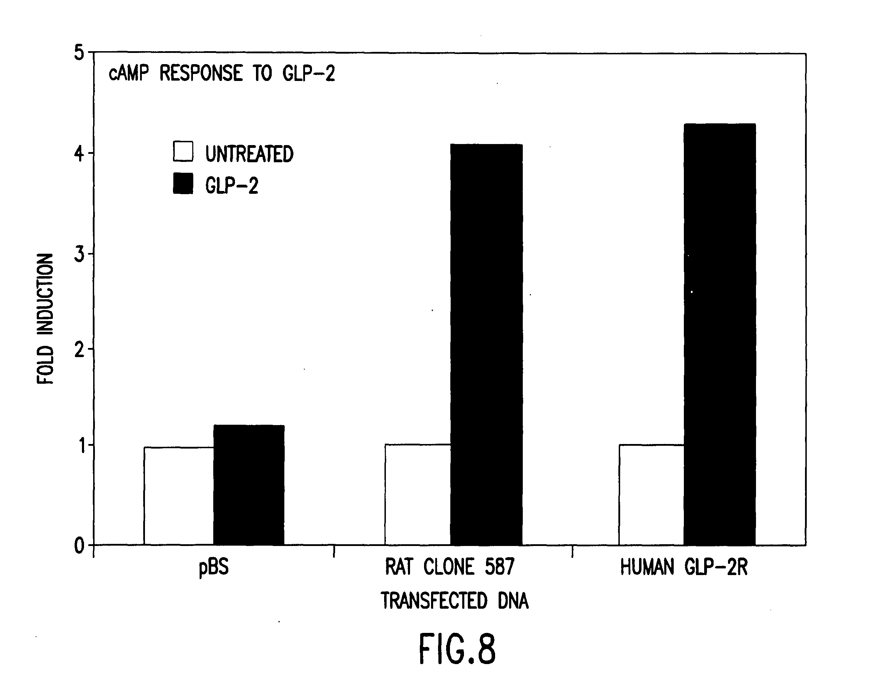

The functional assay was performed as follows: cAMP content for each extract was determined in duplicate by EIA (Enzyme ImmunoAssay) using the Amersham Biotrak cAMP EIA Kit (Amersham 225). Results of the assays, illustrated in Figure 3 and Figure 8, demonstrate the GLP-2 selectivity exhibited by the cloned rat and human receptors. In a similar functional assay used to users binding to the GLP-1 receptor, the expected specificity for GLP-1 was observed.

### EXAMPLE 3

### Isolation Of Human GLP-2 Receptor cDNA

### Medium-stringency hybridization screening of a human hypothalamus cDNA library

One million clones from a λgt10 cDNA library from human hypothalamus (Clontech; Cat. No. 1172a) were screened by plaque lifts on nitrocellulose filters (Amersham; Cat.RPN137E). The probe was prepared by random primer labelling of a DNA fragment containing the complete coding region of rat GLP-2 receptor. The DNA fragment was isolated from clone 587-C1, which contains the complete coding region from SEQ ID NO: 2.

Pre-hybridization and hybridization were each carried out overnight in a hybridization solution consisting of 50% formamide, 5X SSPE, 5X Denhart's solution, 0.5% SDS and salmon sperm DNA (200 mg/ml). After hybridization the filters were washed under the following conditions (medium stringency):
two times at room temperature in 2X SSPE and 0.01% SDS.
two times at 42°C in 2X SSPE and 0.01% SDS.
two times at 42°C in 0.2X SSPE and 0.01% SDS.

The filters were autoradiographed and agar plugs, each containing numerous bacteriophage plaques, were picked from regions on the plates corresponding to positive signals on the filter. From one million cDNA clones sampled in the first round screen, there were identified two positive clones (HHP6-1 and HHP13). On secondary screening only HHP13 turned out positive. Several positive plaques (HHS13) from the HHP13 plate were pooled and taken for tertiary screening. Three single positive plaques from this round of screening were picked (HHT13-1, HHT13-2, HHT13-3).

PCR amplification was then used for partial sequencing of the positive clones. On a lawn of bacterial cells (E.coli C600Hfl), 10 µl of phage suspension from each clone was applied at marked spots. After 5 hr incubation at 37°C, the phage plaques were clearly visible and covered ~1 cm². A portion of each plaque was transferred to 200 µl of water. The samples were incubated in a boiling water bath for 5 min and centrifuged at room temperature for 10 min. One millilitre of sample was used for PCR amplification with two sets of degenerate primers:
M2FS[5'-TTTTTCTAGAASRTSATSTACACNGTSGGCTAC-3'] (SEQ ID NO: 3) and
M7RS[5'-TTTTCTCGAGCCARCARCCASSWRTARTTGGC-3'] (SEQ ID NO: 4); or
C4-4 [5'-AACTACATCCACMKGMAYCTGTTYVYGTCBTTCATSCT-3'] (SEQ ID NO: 5) and
C9-2R [5'-TCYRNCTGSACCTCMYYRTTGASRAARCAGTA-3'] (SEQ ID NO: 8).
The Expand^{™} PCR system from Boehringer Mannheim (Cat. 1681-842) was used under the following conditions:
5 µl of 10x Expand^{™} Buffer 3
7 µl of 2.5mM dNTP mix
1.5 µl of primer M2FS or C4-4
1.5 µl of primer M7RS (with M2FS) or C9-2R (with C4-4)
0.75 µl of Expand PCR enzyme (1 unit)
33.25 µl water and
1 µl DNA.
Reaction conditions were: 32 cycles at 93°C, 1 min; cycles 50°C, 1 min; cycles 45 °C, 1 min; cycles 68°C, 2 min.

M2F/S and M7R/S amplified a DNA fragment of about 300 bp and C4-4 and C92-R amplified a DNA fragment of about 700 bp. The PCR products were purified using the QIAGEN QIAquick PCR purification kit (Cat. 28104) and eluted in 50 µl 10 mM Tris, pH 8.0. Sequence analysis of the products revealed no differences between the templates, as expected from the fact that they represent multiple copies of a single cDNA clone (HHT13).

A number of factors indicate that this clone contains coding sequence of the human GLP-2 receptor. One factor is the degree of sequence similarity. The glucagon receptor cDNA can be used to predict the expected degree of sequence conservation found between rat and human receptors. At the nucleotide level, there is 82.6% identity within the coding regions of the rat and human glucagon receptors. At the amino acid level, there is 80.9% identity and 89.1% amino acid similarity between the glucagon receptors of the two species.

In the case of the human GLP-2 receptor cloned herein, the sequence of the partial human GLP-2 receptor cDNA (HHT13) is highly homologous to rat GLP-2 receptor cDNA at both the nucleotide and amino acid level. SEQ ID NO: 9 shows 87.1% identity with the rat GLP-2 receptor cDNA sequence. The predicted amino acid sequence of this cDNA region has 87.4% identity and 93.2% similarity with the predicted amino acid sequence of the rat GLP-2 receptor. The total predicted length of the rat receptor preprotein is 550 amino acids, suggesting about 44% of the coding region of the human receptor had been identified.

Further evidence supporting this conclusion comes from a comparison of the partial human GLP-2 receptor amino acid sequence with the rat GLP-2 receptor and the 3 next closest family members, shown below:

| Receptor Sequence (amino acid) | Percent Identity with HHT13 | Percent Similarity |
|---|---|---|
| GLP-2 receptor (rat) | 87.4 | 93.2 |
| GLP-1 receptor (rat) | 50.0 | 74.1 |
| Glucagon receptor (rat) | 51.4 | 73.9 |
| GIP receptor (rat) | 50.7 | 70.3 |

These comparisons, together with the benchmark provided by sequence similarities between the rat and human glucagon receptors, provide definitive evidence that the cDNA designated HHT13 represents a fragment of the human counterpart of the rat GLP-2 receptor.

The full amino acid sequence of the human GLP-2 receptor can be obtained by first determining the sequence of the complete cDNA inserts in HHT13-1, HHT13-2 and HHT13-3. By using degenerate primers for PCR amplification and subsequent sequencing, we obtained sequence from only part of each insert. It is possible that these identical clones contain an insert which spans the complete coding sequence of the human GLP-2 receptor preprotein. To determine the complete sequence of the cDNA insert, the clones are grown in large quantity to prepare approximately 20 mg of each equivalent clone. The complete cDNA insert is excised by restriction with Eco RI, and subcloned into pcDNA3 (Invitrogen). Alternatively, two primers from vector sequence flanking the insert are used to amplify the complete cDNA insert using the Expand^{™} PCR system from Boehringer Mannheim (Cat. 1681-842). The amplified cDNA is cut with appropriate restriction enzymes and is subcloned into pcDNA3 (Invitrogen).

If a complete coding sequence is not present in the HHT13 clones, cDNA libraries are screened for additional clones to complete the coding region of human GLP-2 receptor cDNA. Preferably human cDNA libraries (from Stratagene or Clontech) representing the following tissues are used for screening: Human hypothalamus; Human fetal brain; Human duodenum and jejunum; Human stomach; and Human fetal intestine.

Two PCR primers are designed from the sequence of human GLP-2 receptor cDNA already determined. These primers are designed such that they could not amplify any related gene family members other than the GLP-2 receptor cDNA itself. A dilution of the cDNA library stock is used to make library sub-pools such that 50,000 clones are represented in each pool. PCR is conducted with the GLP-2 receptor-specific primers to diagnose pools containing a GLP-2 receptor cDNA clone, using the Expand^{™} PCR system from Boehringer Mannheim (Cat. 1681-842) under the following conditions:
2 µl of 10x Expand^{™} Buffer 1
2.8 µl of 2.5mM dNTP mix
0.6 µl of primer P1
0.6 µl of primer P2
0.3 µl of Expand PCR enzyme (1 unit)
12.7 µl water
1 µl of library pool containing 50,000 clones
Reaction conditions: 32 cycles at 93°C, 40 sec; 50-58°C, 40 sec; 68°C, 40 sec.

Sequence is then obtained from the complete GLP-2 receptor cDNA insert from a positive pool. By using one specific primer and one primer based on vector sequence close to the cloning site, the GLP-2 receptor cDNA insert is directly amplified from clonally impure clone pools, using the Expand^{™} PCR system from Boehringer Mannheim (Catalogue no. 1681-842) most suitably under the following conditions:
2 µl of 10x Expand^{™} Buffer 1
2.8 µl of 2.5mM dNTP mix
0.6 µl of Primer 1
0.6 µl of Primer 2
0.3 µl of Enzyme (1 unit)
12.7 µl water
1 µl of library pool stock
Reaction conditions: 32 cycles at 93°C, 45 sec; 50°C, 45 sec; 68°C, 1 min.
The reaction is run on a preparative agarose gel, and the most prominent band is purified and sequenced. Based on the amplified sequence obtained, additional primers are designed to obtain sequence and clones of complete coding region and clone the complete cDNA

5' RACE and 3' RACE are used to obtain complete coding sequence of the human GLP-2 receptor cDNA. Rapid Amplification of cDNA Ends (RACE) is a procedure routinely used for amplification of DNA sequences from first cDNA strand (easily prepared from mRNA) template between a defined internal site and either 3' or the 5' end of the mRNA. Total or mRNA from different human tissues are commercially available from Clontech. The 3' RACE System (Gibco-BRL Life Technologies; Cat. 18373-019) and 5' RACE System (Cat. 18374-058) kits are used. The manuals of these two products provide detailed protocols. In brief, protocols are as described below.

For the 3' RACE procedure, first strand cDNA synthesis is initiated at the poly (A) tail of mRNA using the adapter primer (provided with system) incorporating a unique sequence for universal PCR amplification of the RACE products. After synthesis of the first strand cDNA from this primer, the original mRNA template is destroyed with RNase H. Amplification is then performed using two primers: one is a gene-specific primer (which will be designed from the available partial cDNA sequence of HHT13); the other is the universal amplification primer provided with the kit. The amplified product is subcloned into a plasmid vector for sequencing.

For the 5' RACE System, the first strand cDNA is synthesized from mRNA using a gene-specific primer (which is based on the available partial cDNA sequence of HHT13) and SuperScript II reverse transcriptase. The original mRNA template is removed by treatment with RNase H. Unincorporated dNTPs, primer, and proteins are separated from cDNA using spin cartridge. A homopolymeric dCTP tail is then added to the 3'- end of the first strand cDNA using TdT enzyme and dCTP nucleotides. PCR amplification is performed using two primers: one is a nested, gene-specific primer designed from the available partial DNA sequence of HHT13; and the other is an "anchor primer" provided with the system. Both primers incorporate restriction sites for subcloning into plasmids and subsequent sequencing.

### Sub-cloning of HHT13 πgt10 clones into pcdna3, their sequencing and expression

### A. Amplification of cDNA inserts with πgt10 primers.

On a lawn of bacterial cells (E.coli C600Hf1), 10 µl of phage resuspension from each clone was placed at marked spots. After 5 hr incubation at 37°C, the phage plaques were clearly visible. The surface of each plaque was transferred to 200 µl of water. The samples were kept in boiling water bath for 5 minutes and centrifuged at room temperature for 10 minutes. 1 µl of sample was used to amplify with a set of πgt10 primers.
GT10-5KXb [5'-GGGTAGTCGGTACCTCTAGAGCAAGTTCAGCC-3'] (SEQ ID NO:18)
vs
GT10-3BXh [5'-ATAACAGAGGATCCTCGAGTATTTCTTCCAG-3'] (SEQ ID NO:19)

The Expand^{™} PCR system from Boehringer Mannheim (Cat. 1681-842) was used under the following conditions:
5 µl of 1Ox Expand^{™} Buffer 3
7 µl of 2.5mM dNTP mix
1.5 µl of primer GT10-5KXb
1.5 µl of primer GT10-3BXh
0.75 µl of Expand PCR enzyme (1 unit)
33.25 µl water
1 µl DNA
Reaction conditions: 5 cycles of 93°C-40 sec; 50 °C- 1 min; 68°C-2 min and 30 cycles of 93°C-40 sec; Ramp to 68°C- I min; 68°C-2 min.

An amplified DNA fragment of about 2200 bp long was seen on the agarose gel from all three clones. The PCR product were purified using the QIAGEN's QIAquick PCR purification kit (Cat. no. 28104) and eluted in 50 µl 10 mM Tris, pH 8.0. The templates were sequenced.

### B. Subcloning into pcDNA3 vector.

The amplified and purified DNA from the three clones was restricted with Kpn I and Xho I and subcloned into pcDNA3 restricted with similar restriction enzymes. The plasmids were named pHHT13-1, pHHT13-2, and pHHT13-3. Plasmids DNAs were prepared using either crude method (alkaline treatment, bacterial DNA precipitation with 3 M KOAc, isopropanol precipitation followed by RNAse treatment and second round of isopropanol precipitation) or plasmid DNA kits from Qiagen Inc. The templates prepared using Qiagen's kits were sequenced.

### C. Functional Assay

Transfections were carried out with each clone, using the rat GLP-2R, 587 clone as a positive control for cAMP response to GLP-2 peptide. Methods for transfection, cell culture and cAMP assay were identical to those described for the functional assay of rat, 587 clone. Results showed that although the positive control gave good cAMP response in COS cells, none of the HHT13 clones gave any cAMP response. As confirmed by sequencing which showed a frame-shift mutation, the functional data suggested that no functional GLP-2R protein was expressed from these cDNA clones.

### D. Comparison of DNA sequences between rat GLP-2R and EEHT13 subclones.

The comparison showed a 2 bp deletion at a position corresponding to nucleotides 389-390 of the rat GLP-2R cDNA, resulting in the loss of nucleotides 374-375 of the human GLP-2R cDNA sequence presented herein.

PCR was used to incorporate two bp of the rat GLP-2R DNA sequence into HHT13-1 DNA at the site of the 2 bp frame-shift deletion identified relative to the rat GLP-2R coding sequence. The following primers were designed from HHT13 DNA sequence to insert two bp
HWBR/2BPI-475F
[5'-ACAGGCATGTCTGGAAGACTTACTCAAGGAACCTTCTGGCAT-3'] (SEQ ID NO:20) HWBR/2BPI-506R
[5'-ATGCCAGAAGGTTCCT1TGAGTAAGTCTTCCAGACATGCCTGT-3'] (SEQ ID NO:21)
HWBR-F7 [5'-TTCCTCTGTGGTACCAAGAGGAATGC-3'] (SEQ ID NO:22) and HWBR-1910R:
[5'-GGTGGACTCGAGGTACCGATCTCACTCTCTTCCAGAATC-3'] (SEQ ID NO:23)

PCR 1: One ng of pHHT13-1 DNA was used as template to do two PCRs with primers, HWBR-F7 vs HWBR/2BPI-506R and HWBR/2BPI-475F vs HWBR-1910R. The Expand^{™} PCR system from Boehringer Mannheim (Cat. 1681-842) was used under the following conditions:
5 µl of Lox Expand^{™} Buffer 3
7 µl of 2.5mM dNTP mix
1.5 µl of primer HWBR-F7 or HWBR/2BPI-475F
1.5 µl of primer HWBR-2BPI-506R or HWBR-1910R
0.75 µl of Expand PCR enzyme (1 unit)
33.25 µl water and
1 µl DNA.
Reaction conditions: 10 cycles of 92°C-40 sec; 48°C-1 mm; 68°C-3 mm and 30 cycles of 92°C-40 sec; 55°C- 40 sec; 68°C-2 mm.

The primers HWBR-F7 and HWBR/2BPI-506R amplified a DNA fragment of 400 bp and HWBR/2BPI-475F and HWBR-1910R amplified a DNA fragment of approximately 1.4 kb on an agarose gel. The two bands were cut out of the agarose gel and purified with Qiaquick gel extraction kit from Qiagen Inc. (Cat no. 28706) and the DNAs were eluted in 50 µl of 10 mM Tris (pH 8.5).

PCR 2 (Extension without primers): Approximately 75 ng of two amplified product from above PCR 1 were mixed and then recombined without primers by extending under the following conditions:
2 µl of 10x Expand^{™} Buffer 1
2.8 µl of 2.5mM dNTP mix
0.3 µl of Expand PCR enzyme (1 unit)
8.9 µl water
6 µl of combined PCR 1 products
Reaction conditions: 15 cycles of 92°C-1 min; 60°C- 5 min; 68°C-3 min.

PCR 3: 1 µl of amplified mix from PCR 2 was used as template to amplify with HWBR-F7 and HWBR- 1910R primers using the following conditions:
10 µl of 10x Expand^{™} Buffer 1
14 µl of 2.5mM dNTP mix
3.0 µl of primer HWBR-F7 or HWBR/2BPI-475F
3.0 µl of primer HWBR/2BPI-506R or HWBR-1910R
1.5 µl of Expand PCR enzyme (1 unit)
67.5 µl water and
1 µl DNA.
Reaction conditions: 30 cycles of 92°C-1 min; 60°C- 1 min; 68°C-2 min.
A DNA fragment of approximately 1.7 kb was amplified as seen on an agarose gel. The PCR product was purified using the QIAGEN's QIAquick PCR purification kit (cat. no. 28104) and eluted in 50 µl of 10 mM Tris, pH 8.0. The purified product was restricted with Kpn I and subcloned into Kpn I-restricted pcDNA3.1(-)/Myc-His A (Invitrogen, Cat. no. V855-20). One clone, named pc3.1/HuGL2R/MH6 (pHuMH6), had the 1.7 kb insert in correct orientation as checked by PCR using vector vs. insert primers.

### Functional assay:

This hybrid clone was compared to rat GLP-2R using the assay described in Example 2. Results showed that the 2 bp *"GA"* replacement into the putative deletion site yielded a clone encoding a functional GLP-2R protein, as shown by the CAMP response to GLP-2 treatment.

### EXAMPLE 6

### Isolation of the Full-Length Human GLP-2 Receptor cDNA

Twenty thousand clones from πgt10 cDNA Library from Human Stomach (Clontech; Cat. HL3O17a) were plated on each of 100 agar 150 mm plates. SM buffer (0.1 M NaCl, 10 mM Mg₂So₄, 35 mM Tris, pH-7.5, 0.01% gelatin) was added to each plate to obtain 100 phage lysates each containing 20,000 (20K) pooled clones. The first fifty 20K phage lysates (20K pools) were screened by PCR using two primers designed from HHT13 DNA sequence. The template DNA from each pool was prepared by boiling phage lysate for 10 minutes and centrifuging for 10 minutes.
HWBR-113F [5'-GTGGAGAGGATTTGTGCAAACATTTC-3'] (SEQ ID NO:24)
HWBR-578R [5'-AGAGACATTTCCAGGAGAAGAATGAG-3'] (SEQ ID NO:25)
1 µl of each 20K pool DNA was diagnosed by PCR with HWBR-113F and HWBR578R primers using the following conditions:
2 µl of 1Ox Expand^{™} Buffer 1
2.8 µl of 2.5mM dNTP mix
0.6 µl of primer HWBR-113F
0.6 µl of primer HWBR-578R
0.3 µl of Expand PCR enzyme (1 unit)
12.7 µl water
1 µl 20K pool DNA
Reaction conditions: 35 cycles of 92°C-40s.; 60°C-40s.; 68°C-1 min.

A DNA fragment of approximately 450 bp was seen in amplification of templates from two pools (HST 19 and HST 38).

### B. Screening of clones from two positive pools: HST 19 and HST 38.

40,000 clones plated from each of two positive 20K pools were screened by plaque lifts on nitrocellulose filters (Amersham; Cat.RPN137E). The probe was prepared by random primer labelling a DNA fragment from pHHT13-1.
1. The filters were pre-hybridized and hybridized at 42°C overnight. Hybridization solution consisted of 50% formamide, 5X SSPE, 5X Denhart's solution, 0.5% SDS and salmon sperm DNA (200 mg/ml).
2. After hybridization the filters were washed under the following conditions:
   - two times at room temperature in 2X SSPE and 0.01% SDS;
   - two times at 42°C in 2X SSPE and 0.01% SDS; and
   - two times at 50°C in 0.1X SSPE and 0.01% SDS.
3. The filters were autoradiographed and the regions on the plates matching to positive signals were isolated. One positive clone (HST 38-4-30) was isolated from HST 38 pool. 450 bp DNA fragment was amplified from the positive clone by using primers HWBR-113F and HWBR-578R and sequenced. The sequence clearly showed that the plasmid contain 2 bp (A G) at position 373-374 of HHT13 DNA sequence.

The complete insert of clone HST 38-4-30 was amplified using λgt10 primers as described in Example 1. PCR amplified a DNA fragment of approximately 1.4 kb. The amplified DNA was purified and sequenced.

### EXAMPLE 7

### Reconstruction of a clone of full-length functional human GLP-2R cDNA and functional assay

A 700 bp fragment obtained by Kpn I and Pvu II restriction digest of the amplified DNA from clone UST 38-4-30, and 1.4 kb DNA fragment from Xho I and Pvu II restricted pHHT13-1 DNA were subcloned into Kpn I and Xho I restricted pcDNA3 in a three-way ligation. The new plasmid construct was called pc3/HuGL2R-2. In this manner the full length sequence of the human GLP-2 receptor was obtained.

### Functional Assay:

The new clone was compared to the rat GLP-2R clone 587 as described previously above. Results showed that the clone encoded a functional human GLP-2R protein, which led to cAMP production in COS cells in response to GLP-2 treatment (Figure 8).

### EXAMPLE 8 Antibodies directed to the GLP-2 receptor

### 1. Antipeptide antibodies

Antipeptide antibodies were raised in rabbits against an N-terminal peptide (QTRENTTDIWQDESE) (SEQ ID NO:26), a C-terminal peptide (SEGDGSETLQKLR) (SEQ ID NO:27) and extracellular loop 1(SHNSUSKRPDDESG) (SEQ ID NO:28) of the rat GLP-2 receptor.
Immunocytochemical analysis of serum produced as above confirmed that serum contained antibodies directed to the GLP-2 receptor which do not cross react with the rat GLP-1 receptor.

### 2. Antibodies to a GLP-2 receptor raised against a fusion protein

Polynucleotide encoding the C-terminal region of rat GLP-2 receptor (amino acids 444-550) was spliced to the C-terminus of glutathione 5-transferase (GST) in pGEX-2T and expressed in E.coli strain SUREI. Protein was purified using affinity chromatography using the above GLP-2 C-terminal fragment fused to the C-terminus of maltose binding protein. Protease degradation was minimized by using a cocktail or protease inhibitors (Boeringer Mannheim).

Antibodies were raised generally according to the method disclosed in Antibodies: A laboratory manual, Harlow and Lane, Cold Spring Harbor Laboratory, 1988. Briefly, the GLP-2-GST fusion protein was used to raise antibodies in rabbits as follows. Initial injection was with 100 µg of fusion protein in complete Freund's adjuvant at multiple sites, intramuscularly and subcutaneously. Booster injections were made at multiple sites intramuscularly with 100 µg of fusion protein in incomplete Freund's adjuvant at days 14, 21, 42 and 56.

Antisera was affinity purified using a GLP-2-MBP fusion protein affinity column. Immunocytochemical analysis confirmed that these antibodies specifically recognize the GLP-2 receptor.

### EQUIVALENTS

The foregoing written specification is considered to be sufficient to enable one skilled in the art to practice the invention. Indeed, various modifications of the above-described makes for carrying out the invention which are obvious to those skilled in the field of biochemistry, molecular biology or related fields are intended to be within the scope of the following claims.

## Claims

1. An isolated polynucleotide which encodes a GLP-2 receptor selected from:
(a) a human GLP-2 receptor comprising the amino acid sequence of amino acids 67-553 of SEQ ID NO: 12;
(b) a rat GLP-2 receptor comprising the amino acid sequence of amino acids 67-550 of SEQ ID NO: 2;
(c) a mammalian homolog having at least 80% sequence identity to (a) or (b) which exhibits the functional characteristic of selectively binding GLP-2, and
(d) a variant of (a), (b), or (c) which exhibits the functional characteristic of selectively binding GLP-2, the variant comprising from 1 to 20 amino acid substitution(s), wherein the substitution(s) is/are (i) a conservative substitution(s) when at a position conserved across receptors (a) and (b), and/or (ii) a conservative or non-conservative substitution(s) when at a position not conserved across receptors (a) and (b).

2. An isolated polynucleotide which encodes a GLP-2 receptor according to claim 1 wherein said GLP-2 receptor is a human GLP-2 receptor comprising the amino acid sequence of amino acids 67-553 of SEQ ID NO: 12.

3. An isolated polynucleotide which encodes a GLP-2 receptor according to claim 1 wherein said GLP-2 receptor is a rat GLP-2 receptor comprising the amino acid sequence of amino acids 67-550 of SEQ ID NO: 2.

4. An isolated polynucleotide according to claim 1, comprising nucleotides 320-1780 of SEQ ID NO: 11.

5. An isolated polynucleotide according to claim 1, having nucleotides 335-1786 of SEQ ID NO: 1.

6. An isolated polynucleotide according to claim 1, wherein said polynucleotide encodes a Glu85 variant of said human GLP-2 receptor, as shown in SEQ ID NO: 12.

7. An isolated polynucleotide according to claim 1, wherein said polynucleotide encodes for the human GLP-2 receptor and said polynucleotide encodes for the amino acid sequence of amino acids 1-553 of SEQ ID NO: 12.

8. An isolated polynucleotide according to claim 1, wherein said polynucleotide encodes for the human GLP-2 receptor and said polynucteotide encodes for the amino acid sequence of amino acids 26-553 of SEQ ID NO: 12

9. An isolated polynucleotide according to claim 1, wherein said polynucleotide encodes for the human GLP-2 receptor and said polynucleotide encodes for the amino acid sequence which is at least 95% identical to amino acids 26-553 of SEQ ID NO: 12.

10. An isolated polynucleotide which hybridizes under conditions of high stringency with a polynucleotide according to any of claims 1 - 9, or with the complement of said polynucleotide.

11. An oligonucleotide comprising at least 15 nucleotides and having a polynucleotide sequence that hybridizes under conditions of high stringency to a polynucleotide according to any of claims 1 - 10 or with the complement of said polynucleotide.

12. An oligonucleotide according to claim 11, wherein the nucleotide sequence of the oligonucleotide is identical to a region of the polynucleotide defined in claims 4 or 5 or with the complement of said polynucleotide.

13. An oligonucleotide according to claim 11, wherein the nucleotide sequence of the oligonucleotide is identical to a region common to the polynucleotides defined in claims 4 or 5, or with the complement of said polynucleotide.

14. In labeled form, a polynucleotide selected from a polynucleotide as defined in any of claims 1 - 10.

15. A recombinant polynucleotide comprising a GLP-2 receptor-encoding polynucleotide as defined in any of claims 1 - 10, and expression controlling elements linked operably therewith to drive expression thereof.

16. A cell that has been genetically engineered by the incorporation expressibly therein of a polynucleotide according to any one of claims 1 - 10.

17. The cell according to claim 16, which is a mammalian cell.

18. An antibody which selectively binds to a GLP-2 receptor coded for by the polynucleotide according to any one of claims 1 - 5, 7 - 10.

19. A recombinant GLP-2 receptor selected from:
(a) a human GLP-2 receptor comprising the amino acid sequence of amino acids 67-553 of SEQ ID NO: 12;
(b) a rat GLP-2 receptor comprising the amino acid sequence of amino acids 67-550 of SEQ ID NO: 2;
(c) a mammalian homolog having at least 80% sequence identity to (a) or (b) which exhibits the functional characteristic of selectively binding GLP-2,
(d) a variant of (a), (b), or (c) which exhibits the functional characteristic of selectively binding GLP-2, the variant comprising from 1 to 20 amino acid substitution(s), wherein the substitution(s) is/are (i) a conservative substitution when at a position conserved across receptors (a) and (b), and/or (ii) a conservative or non-conservative substitution(s) when at a position not conserved across receptors (a) and (b), and
(e) a chimeric form of (a), (b), (c) or (d) comprising one or more domains from a G-protein coupled receptor other than the GLP-2 receptor wherein the domains are a transmembrane domain, or an intracellular domain.

20. A recombinant GLP-2 receptor according to claim 19, wherein said GLP-2 receptor is human GLP-2 receptor comprising the amino acid sequence of amino acids 67-553 of SEQ ID NO: 12.

21. A recombinant GLP-2 receptor according to claim 19, wherein said GLP-2 receptor is rat GLP-2 receptor comprising the amino acid sequence of amino acid 67-550 of SEQ ID NO: 2.

22. A recombinant GLP-2 receptor according to claim 19, wherein the GLP-2 receptor is a Glu85 variant of the human GLP-2 receptor as shown in SEQ ID NO: 12.

23. A fragment of a GLP-2 receptor as defined in claims 19 - 22, wherein said fragment comprises an amino acid sequence constituting an intracellular C-terminal domain of a GLP-2 receptor.

24. A fragment of a GLP-2 receptor according to claim 23, comprising at least 10 amino acid residues within the region of SEQ ID NO: 12 corresponding to amino acids selected from the group consisting of amino acids 65-180, 363-385 and 442-533.

25. An antibody which selectively binds to a GLP-2 receptor according to any one of claims 19 - 21.

26. A cell membrane preparation derived from a cell according to claim 16 or claim 17.

27. A method for identifying GLP-2 receptor ligands, which comprises the step of:
(1) incubating a candidate ligand with a cell as defined in claim 16 or 17 or with a membrane preparation obtained therefrom, and then
(2) determining whether binding between the GLP-2 receptor and the candidate ligand has occurred.

28. A method for identifying GLP-2 receptor ligands, comprising the steps of:
a. identifying a cell expressing a functional GLP-2 receptor, said cell containing a recombinant DNA molecule encoding for the GLP-2 receptor,
b. incubating a candidate ligand with the cell that expresses a functional GLP-2 receptor, or with a membrane preparation derived from said cell; and
c. determining whether binding between the GLP-2 receptor and the ligand has occurred.

29. A method according to claim 28, wherein the candidate ligand is incubated with a cell that produces a functional GLP-2 receptor, and the determination whether binding has occurred between the GLP-2 receptor and the ligand candidate is achieved by measuring change in the intracellular cAMP level, an increase in the cAMP level indicating that the candidate ligand is a GLP-2 agonist.

30. An isolated polynucleotide according to claim 1, comprising a nucleotide sequence having at least 80% sequence identity to SEQ ID NO: 1, wherein said polynucleotide encodes a mammalian GLP-2 receptor that exhibits the functional characteristics of selectively binding GLP-2.

31. The isolated polynucleotide according to claim 1, wherein said nucleotide sequence has at least 90% sequence identity to SEQ ID NO: 1, wherein said polynucleotide encodes a mammalian GLP-2 receptor that exhibits the functional characteristics of selectively binding GLP-2.

32. The isolated polynucleotide according to claim 1, wherein said nucleotide sequence has at least 95% sequence identity to SEQ ID NO: 1, wherein said polynucleotide encodes a mammalian GLP-2 receptor that exhibits the functional characteristics of selectively binding GLP-2.

## Patentansprüche

1. Isoliertes Polynucleotid, welches einen GLP-2-Rezeptor kodiert, ausgewählt aus:
(a) einem Human-GLP-2-Rezeptor, aufweisend die Aminosäuresequenz von Aminosäuren 67-553 der SEQ ID Nr.: 12;
(b) einen Ratten-GLP-2-Rezcptor, aufweisend die Aminosäuresequenz von Aminosäuren 67-550 der SEQ ID Nr.: 2;
(c) ein Mammalia-Homolog mit mindestens 80% Sequenzidentität zu (a) oder (b), das das funktionelle Merkmal des selektiven Bindens von GLP-2 zeigt, und
(d) eine Variante von (a), (b) oder (c), die das funktionelle Merkmal des selektiven Bindens von GLP-2 zeigt, wobei die Variante von 1 bis 20 Aminosäuren-Substitution(en) aufweist, worin der/die Substitution(en) ist/sind: (i) eine konservative Substitution(en), wenn dieses sich an einer über den Rezeptoren (a) und (b) konservierten Position befinden, und/oder (ii) eine/mehrere konservative oder nichtkonservative Substitution(en), wenn diese sich an einer nichtkonservierten Position über den Rezeptoren (a) und (b) befinden.

2. Isoliertes Polynucleotid nach Anspruch 1, welches einen GLP-2-Rezeptor kodiert, wobei der GLP-2-Rezeptor ein Human-GLP-2-Rezeptor ist, aufweisend die Aminosäuresequenz von Aminosäuren 67-553 der SEQ ID Nr.: 12.

3. Isoliertes Polynucleotid nach Anspruch 1, welches einen GLP-2-Rezeptor kodiert, wobei der GLP-2-Rezeptor ein Ratten-GLP-2-Rezeptor ist, aufweisend die Aminosäuresequenz von Aminosäuren 67-550 der SEQ ID Nr.: 2.

4. Isoliertes Polynucleotid nach Anspruch 1, aufweisend die Nucleotide 320 bis 1780 der SEQ ID Nr.: 11.

5. Isoliertes Polynucleotid nach Anspruch 1 mit den Nucleotiden 335 bis 1786 der SEQ ID Nr.: 1.

6. Isoliertes Polynucleotid nach Anspruch 1, wobei das Polynucleotid eine Glu85-Variante des Human-GLP-2-Rezeptors kodiert, wie er in SEQ ID Nr.: 12 gezeigt ist.

7. Isoliertes Polynucleotid nach Anspruch 1, wobei das Polynucleotid für den Human-GLP-2-Rezeptor kodiert und das Polynucleotid für die Aminosäuresequenz von Aminosäuren 1-553 der SEQ ID Nr.: 12 kodiert.

8. Isoliertes Polynucleotid nach Anspruch 1, wobei das Polynucleotid für den Human-GLP-2-Rezeptor kodiert und das Polynucleotid für die Aminosäuresequenz von Aminosäuren 26-553 der SEQ ID Nr.: 12 kodiert.

9. Isoliertes Polynucleotid nach Anspruch 1, wobei das Polynucleotid für den Human-GLP-2-Rezeptor kodiert und das Polynucleotid für die Aminosäuresequenz kodiert, die zu mindestens 95 % mit den Aminosäuren 26-553 der SEQ ID Nr.: 12 identisch ist.

10. Isoliertes Polynucleotid, welches unter Bedingungen hoher Stringenz mit einem Polynucleotid nach einem der Ansprüche 1 bis 9 hybridisiert oder mit dem Komplement des Polynucleotids.

11. Oligonucleotid, aufweisend mindestens 15 Nucleotide und mit einer Polynucleotidsequenz, welche unter Bedingungen hoher Stringens mit einem Polynucleotid nach einem der Ansprüche 1 bis 10 hybridisiert oder mit dem Komplement des Polynucleotids.

12. Oligonucleotid nach Anspruch 11, wobei die Nucleotidsequenz des Oligonucleotids mit einer Region des Polynucleotids identisch ist, das in Ansprüchen 4 oder 5 festgelegt ist, oder mit dem Komplement des Polynucleotids.

13. Oligonucleotid nach Anspruch 11, wobei die Nucleotidsequenz des Oligonucleotids mit einer Region identisch ist, die mit dem Polynucleotiden gemeinsam ist, die in Ansprüchen 4 oder 5 festgelegt sind, oder mit dem Komplement des Polynucleotids.

14. Markierte Form eines Polynucleotids, ausgewählt aus einem Polynucleotid nach einem der Ansprüche 1 bis 10.

15. Rekombinantes Polynucleotid, aufweisend ein GLP-2-Rezeptor kodierendes Polynucleotid nach einem der Ansprüche 1 bis 10 sowie die Expression kontrollierende Elemente, die funktionsfähig damit verknüpft sind, um deren Expression zu steuern.

16. Zelle, die genetisch bearbeitet ist, indem exprimierbar darin ein Polynucleotid nach einem der Ansprüche 1 bis 10 eingebaut ist.

17. Zelle nach Anspruch 16, die eine Mammalia-Zelle ist.

18. Antikörper, der selektiv an einen GLP-2-Rezeptor bindet, kodiert durch das Polynucleotid nach einem der Ansprüche 1 bis 5, 7 bis 10.

19. Rekombinanter GLP-2-Rezeptor, ausgewählt aus:
(a) einem Human-GLP-2-Rezeptor, aufweisend die Aminosäuresequenz von Aminosäuren 67-553 der SEQ ID Nr.: 12;
(b) einem Ratten-GLP-2-Rezeptor, aufweisend die Aminosäuresequenz von Aminosäuren 67-550 der SEQ ID Nr.: 2;
(c) ein Mammalia-Homolog mit mindestens 80% Sequenzidentität zu (a) oder (b), welches das funktionelle Merkmal des selektiven Bindens von GLP-2 zeigt;
(d) eine Variante von (a), (b) oder (c), die das funktionelle Merkmal des selektiven Bindens von GLP-2 zeigt, wobei die Variante von 1 bis 20 Aminosäure-Substitution(en) aufweist, worin die Substitution(en) ist/sind: (i) eine konservative Substitution, wenn sie sich an einer Position befindet, die über den Rezeptoren (a) und (b) erhalten ist, und/oder (ii) eine/mehrere konservative oder nichtkonservative Substitution(en), wenn sie sich an einer Position befindet, die nicht über den Rezeptoren (a) und (b) erhalten ist, und
(e) eine chimäre Form von (a), (b), (c) oder (d), aufweisend eine oder mehrere Domänen von einem G-Protein-gekoppelten Rezeptor außer dem GLP-2-Rezeptor, worin die Domänen eine Transmembran-Domäne sind oder eine interzelluläre Domäne sind.

20. Rekombinanter GLP-2-Rezeptor nach Anspruch 19, wobei der GLP-2-Rezeptor ein Human-GLP-2-Rezeptor ist, aufweisend die Aminosäuresequenz von Aminosäuren 67-553 der SEQ ID Nr.: 12.

21. Rekombinanter GLP-2-Rezeptor nach Anspruch 19, wobei der GLP-2-Rezeptor ein Ratten-GLP-2-Rezeptor ist, aufweisend die Aminosäuresequenz von Aminosäuren 67-550 der SEQ ID Nr.: 2.

22. Rekombinanter GLP-2-Rezeptor nach Anspruch 19, wobei der GLP-2-Rezeptor eine Glu85-Variante des Human-GLP-2-Rezeptors ist, wie in SEQ ID Nr.: 12 gezeigt ist.

23. Fragment eines GLP-2-Rezeptors nach Anspruch 19 bis 22, wobei das Fragment eine Aminosäuresequenz aufweist, die eine interzelluläre C-Terminal-Domäne eines GLP-2-Rezeptors darstellt.

24. Fragment eines GLP-2-Rezeptors nach Anspruch 23, aufweisend mindestens 10 Aminosäure-Reste in der Region der SEQ ID Nr.: 12 entsprechend den Aminosäuren, die ausgewählt sind aus der Gruppe, bestehend aus Aminosäuren 65-180, 363-385 und 442-533.

25. Antikörper, der selektiv an einen GLP-2-Rezeptor nach einem der Ansprüche 19 bis 21 bindet.

26. Zellmembranpräparat, deriviert von einer Zelle nach Anspruch 16 oder Anspruch 17.

27. Verfahren zum Identifizieren von GLP-2-Rezeptorliganden, welches Verfahren die Schritte umfasst:
(1) Inkubieren eines Ligandenkandidaten mit einer Zelle nach Anspruch 16 oder 17 oder mit einem daraus erhaltenen Membranpräparat und anschließend
(2) Bestimmen, ob ein Binden zwischen dem GLP-2-Rezeptor und dem Ligandenkandidaten erfolgt ist.

28. Verfahren zum Identifizieren von GLP-2-Rezeptorliganden, umfassend die Schritte:
a. Identifizieren einer Zelle, die einen funktionellen GLP-2-Rezeptor exprimiert, wobei die Zelle, die ein rekombinantes DNA-Molekül enthält, für den GLP-2-Rezeptor kodiert,
b. Inkubieren eines Ligandenkandidaten mit der Zelle, die einen funktionellen GLP-2-Rezeptor exprimiert, oder mit einem Membranpräparat, das von dieser Zelle deriviert ist, und
c. Bestimmen, ob ein Binden des GLP-2-Rezeptors und des Liganden erfolgt ist.

29. Verfahren nach Anspruch 28, wobei der Ligandenkandidat mit einer Zelle inkubiert ist, die einen funktionellen GLP-2-Rezeptor erzeugt und die Bestimmung, ob ein Binden zwischen dem GLP-2-Rezeptor und den Ligandenkadidaten erreicht worden ist, indem die Änderung in der interzellulären cAMP-Menge gemessen wird, wobei eine Zunahme der cAMP-Menge zeigt, dass der Ligandenkandidat ein GLP-2-Agonist ist.

30. Isoliertes Polynucleotid nach Anspruch 1, aufweisend eine Nucleotidsequenz mit mindestens 80% Sequenz-Identität zur SEQ ID Nr.: 1, wobei das Polynucleotid einen Mammalia-GLP-2-Rezeptor kodiert, der die funktionellen Merkmale des selektiven Bindens von GLP-2 zeigt.

31. Isoliertes Polynucleotid nach Anspruch 1, wobei die Nucleotidsequenz mindestens eine Sequenzidentität zur SEQ ID Nr. 1 von 90% hat, wobei das Polynucleotid einen Mammalia-GLP-2-Rezeptor kodiert, der die funktionellen Merkmale des selektiven Bindens von GLP-2 zeigt.

32. Isoliertes Polynucleotid nach Anspruch 1, wobei die Nucleotidsequenz mindestens 95 % Sequenzidentität zur SEQ ID Nr.: 1 hat, wobei das Polynucleotid einen Mammalia-GLP-2-Rezeptor kodiert, der die funktionellen Merkmale des selektiven Bindens von GLP-2 zeigt.

## Revendications

1. Polynucléotide isolé qui code un récepteur de GPL-2 choisi dans le groupe consistant en:
(a) un récepteur de GPL-2 humain comprenant la séquence aminoacidique d'acides aminés 67-553 de la SEQ ID n° 12;
(b) un récepteur de GPL-2 de rat comprenant la séquence aminoacidique d'acides aminés 67-550 de la SEQ ID n° 2;
(c) un homologue mammifère présentant une identité de séquence d'au moins 80% vis-à-vis de (a) ou vis-à-vis de (b) qui possède la caractéristique fonctionnelle de se lier sélectivement à GPL-2, et
(d) un variant de (a), (b), ou (c) qui possède les caractéristiques fonctionnelles de se lier sélectivement à GPL-2, le variant comprenant de 1 à 20 substitutions d'acides aminés, dans lesquelles la ou les substitutions est/sont (i) une ou plusieurs substitutions conservatives lorsqu'à une position conservée sur les récepteurs (a) et (b), et/ou (ii) une ou plusieurs substitutions conservatives ou non conservatives lorsqu'à une position non conservée sur les récepteurs (a) et (b).

2. Polynucléotide isolé qui code un récepteur de GPL-2 selon la revendication 1, dans lequel ledit récepteur de GPL-2 est un récepteur de GPL-2 humain comprenant la séquence aminoacidique d'acides aminés 67-553 de la SEQ ID n° 12.

3. Polynucléotide isolé qui code un récepteur de GPL-2 selon la revendication 1, dans lequel ledit récepteur de GPL-2 est un récepteur de GPL-2 de rat comprenant la séquence aminoacidique d'acides aminés 67-550 de la SEQ ID n° 2.

4. Polynucléotide isolé selon la revendication 1, comprenant les nucléotides 320-1780 de la SEQ ID n° 11.

5. Polynucléotide isolé selon la revendication 1, portant les nucléotides 335-1786 de la SEQ ID n° 1.

6. Polynucléotide isolé selon la revendication 1, dans lequel ledit polynucléotide code un variant Glu85 dudit récepteur de GPL-2 humain, comme montré dans SEQ ID n° 12.

7. Polynucléotide isolé selon la revendication 1, dans lequel ledit polynucléotide code pour le récepteur de GPL-2 humain et ledit polynucléotide code pour la séquence aminoacidique d'acides aminés 1-553 de la SEQ ID n° 12.

8. Polynucléotide isolé selon la revendication 1, dans lequel ledit polynucléotide code pour le récepteur de GPL-2 humain et ledit polynucléotide code pour la séquence aminoacidique d'acides aminés 26-553 de la SEQ ID n° 12.

9. Polynucléotide isolé selon la revendication 1, dans lequel ledit polynucléotide code pour le récepteur de GPL-2 humain et ledit polynucléotide code pour la séquence aminoacidique qui est identique à au moins 95% aux acides aminés 26-553 de la SEQ ID n° 12.

10. Polynucléotide isolé qui s'hybride dans des conditions de haute stringence à un polynucléotide selon l'une quelconque des revendications 1 à 9 ou au complément dudit polynucléotide.

11. Oligonucléotide comprenant au moins 15 nucléotides et ayant une séquence polynucléotidique qui s'hybride dans des conditions de haute stringence à un polynucléotide selon l'une quelconque des revendications 1 à 10 ou au complément dudit polynucléotide.

12. Oligonucléotide selon la revendication 11, dans lequel la séquence nucléotidique de l'oligonucléotide est identique à une région du polynucléotide défini dans la revendication 4 ou 5 ou au complément dudit polynucléotide.

13. Oligonucléotide selon la revendication 11, dans lequel la séquence nucléotidique de l'oligonucléotide est identique à une région commune aux polynucléotides définis dans la revendication 4 ou 5, ou au complément dudit polynucléotide.

14. Forme marquée d'un polynucléotide choisi parmi un polynucléotide telle que défini dans l'une quelconque des revendications 1 à 10.

15. Polynucléotide recombinant comprenant un polynucléotide codant pour un récepteur de GPL-2 tel que défini dans l'une quelconque des revendications 1 à 10 et des éléments contrôlant l'expression liée fonctionnellement à celui-ci pour en diriger l'expression.

16. Cellule qui a été créée par génie génétique par incorporation incluant la caractéristique fonctionnelle pour diriger l'expression d'un polynucléotide selon l'une quelconque des revendications 1 à 10.

17. Cellule selon la revendication 16, qui est une cellule mammifère.

18. Anticorps qui se lie sélectivement à un récepteur de GPL-2 codé par le polynucléotide selon l'une quelconque des revendications 1 à 5, 7 à 10.

19. Récepteur de GPL-2 recombinant choisi dans le groupe consistant en:
(a) un récepteur de GPL-2 humain comprenant la séquence aminoacidique d'acides aminés 67-553 de la SEQ ID n° 12;
(b) un récepteur de GPL-2 de rat comprenant la séquence aminoacidique d'acides aminés 67-550 de la SEQ ID n° 2;
(c) un homologue mammifère présentant une identité de séquence d'au moins 80% vis-à-vis de (a) ou de (b) qui possède la caractéristique fonctionnelle de se lier sélectivement à GPL-2,
(d) un variant de (a), (b) ou (c) qui possède la caractéristique fonctionnelle de se lier sélectivement à GPL-2, le variant comprenant de 1 à 20 substitutions d'acides aminés, dans lequel la ou les substitutions est/sont (i) une substitution conservative lorsqu'à une position conservée sur les récepteurs (a) et (b), et/ou (ii) une ou plusieurs substitutions conservatives ou non conservatives lorsqu'à une position non conservée sur les récepteurs (a) et (b), et
(e) une forme chimère de (a), (b), (c), ou (d) comprenant un ou plusieurs domaines provenant d'un récepteur couplé à une protéine-G autre que le récepteur de GPL-2, dans laquelle les domaines sont un domaine transmembranaire ou un domaine intracellulaire.

20. Récepteur de GPL-2 recombinant selon la revendication 19, dans lequel ledit récepteur de GPL-2 est un récepteur de GPL-2 humain comprenant la séquence aminoacidique d'acides aminés 67-553 de la SEQ ID n° 12.

21. Récepteur de GPL-2 recombinant selon la revendication 19, dans lequel ledit récepteur de GPL-2 est un récepteur de GPL-2 de rat comprenant la séquence aminoacidique d'acides aminés 67-550 de la SEQ ID n° 2.

22. Récepteur de GPL-2 recombinant selon la revendication 19, dans lequel ledit récepteur de GPL-2 est un variant Glu85 du récepteur de GPL-2 humain, comme montré dans la SEQ ID n° 12.

23. Fragment d'un récepteur de GPL-2 tel que défini dans les revendications 19 à 22, dans lequel ledit fragment comprend une séquence aminoacidique constituant un domaine C-terminal intracellulaire d'un récepteur de GPL-2.

24. Fragment de récepteur de GPL-2 selon la revendication 23, comprenant au moins 10 résidus aminoacidiques à l'intérieur de la région de la SEQ ID n° 12 correspondant aux acides aminés choisis dans le groupe constitué des acides aminés 67-180, 363-385 et 442-533.

25. Anticorps qui se lie sélectivement à un récepteur de GPL-2 selon l'une quelconque des revendications 19 à 21.

26. Préparation de membrane cellulaire dérivée d'une cellule selon la revendication 16 ou la revendication 17.

27. Procédé d'identification de ligands de récepteur de GPL-2, qui comprend l'étape consistant à:
(1) incuber un ligand candidat avec une cellule tel que défini dans la revendication 16 ou 17 ou avec une préparation membranaire obtenue à partir de celle-ci, puis
(2) à déterminer si la liaison entre le récepteur de GPL-2 et le ligand candidat se produit.

28. Procédé d'identification de ligands de récepteur de GPL-2, comprenant les étapes consistant à:
a. identifier une cellule exprimant un récepteur de GPL-2 fonctionnel, ladite cellule contenant une molécule d'ADN recombinant codant pour le récepteur de GPL-2,
b. incuber un ligand candidat avec la cellule qui exprime un récepteur de GPL-2 fonctionnel ou avec une préparation membranaire dérivée de ladite cellule; et
c. à déterminer si la liaison entre le récepteur de GPL-2 et le ligand se produit.

29. Procédé selon la revendication 28, dans lequel le ligand candidat est incubé avec une cellule qui produit un récepteur de GPL-2 fonctionnel et la détermination si la liaison s'est produite entre le récepteur de GPL-2 et le ligand candidat est obtenue en mesurant un changement dans le niveau de l'AMPc intracellulaire, une augmentation du niveau de l'AMPc indiquant que le ligand candidat est un agoniste de GPL-2.

30. Polynucléotide isolé selon la revendication 1, comprenant une séquence nucléotidique présentant une identité de séquence d'au moins 80% avec la SEQ ID n° 1, dans lequel ledit polynucléotide code pour un récepteur de GPL-2 mammifère qui possède la caractéristique fonctionnelle de se lier sélectivement à GPL-2.

31. Polynucléotide isolé selon la revendication 1, dans lequel ladite séquence nucléotidique présente une identité de séquence d'au moins 90% avec la SEQ ID n° 1, dans lequel ledit polynucléotide code pour un récepteur de GPL-2 mammifère qui possède la caractéristique fonctionnelle de se lier sélectivement à GPL-2.

32. Polynucléotide isolé selon la revendication 1, dans lequel ladite séquence nucléotidique présente une identité de séquence d'au moins 95% avec la SEQ ID n° 1, dans lequel ledit polynucléotide code pour un récepteur de GPL-2 mammifère qui possède la caractéristique fonctionnelle de se lier sélectivement à GPL-2.
